# EUROPEAN PATENT SPECIFICATION

(11) **EP 2 471 784 B1**
(45) Date of publication and mention of the grant of the patent: **08.07.2015**
(21) Application number: 12161958.9
(22) Date of filing: 15.10.2008
(51) Int. Cl.: C07D 333/26, C09B 49/00, C09B 49/06

(54) **Novel thiophene-based dye and preparation thereof**
Neuer Farbstoff auf Thiophenbasis und Herstellung davon
Nouveau colorant à base de Thiophène et sa préparation

(30) Priority: 15.10.2007 KR 20070103629; 26.12.2007 KR 20070137908; 25.04.2008 KR 20080038731; 30.06.2008 KR 20080062482
(43) Date of publication of application: 04.07.2012
(62) Divisional of application: 08839120.6
(73) Proprietor: Dongjin Semichem Co., Ltd., Seo-gu Incheon 404-205 (KR)
(72) Inventor: Bae, Ho-Gi, 451-781 Pyeongtaek-city Gyeonggi-do (KR); Lee, Chong-chan, 363-883 Chungcheongbuk-do (KR); Kim, Jong-bok, 138-172 Seoul (KR); Moon, Hyoung-don, 447-270 Osan City Gyeonggi-do (KR); Park, Tae-jin, 405-759 Incheon (KR); Baek, Jong-hyub, 139-224 Seoul (KR); Yang, Hoe-taek, 445-923 Hwaseong-city Gyeonggi-do (KR); An, Hyun-cheol, 445-931 Hwaseong-city Gyeonggi-do (KR)
(74) Representative: Bockhorni & Kollegen

(56) References cited:
- CN-A- 101 029 182

## Description

The present invention relates to a thiophene-based dye and a preparation thereof which is used for a dye-sensitized solar cell.

### [Background Art]

Since a research team of Michael Grätzel at the Ecole Polytechnique Federale de Lausanne (EPFL) in Switzerland developed a dye-sensitive nano particle titanium dioxide solar cell in 1991, lots of studies have been conducted on the area. The dye-sensitized solar cell provides higher efficiency and requires significantly lower manufacturing costs than an existing silicon solar cell does, and can possibly replace an existing amorphous silicon solar cell. Unlike a silicon solar cell, the dye-sensitized solar cell is a photoelectrochemical solar cell which includes a dye molecule absorbing visible rays to generate an electron-hole pair and a transition metal oxide transferring a generated electron, as main materials.

Ruthenium metal complexes which are widely used in the dye-sensitized solar cell cost too much while providing high photoelectric conversion efficiency.

Recently, it has been found that a metal-free organic dye, which has good properties from the perspective of the light absorption efficiency, stable oxidation-reduction reaction and charge-transfer (CT) absorption within molecules, can be used in the solar cell instead of expensive ruthenium metal complexes. Thus, there have been full-scale studies for the metal-free organic dye.

The organic dye generally includes a structure of electron donor-electron acceptor end groups which are connected by a π-coupling unit. In most of organic dyes, an amine derivative acts as an electron donor and 2-cyanoacrylic acid or rhodanine end groups act as an electron acceptor, both of which are connected by π-coupling system such as metaphosphorus unit or thiophene chain.

For example, CN 101 029 182 A discloses an organic dye of the following formula

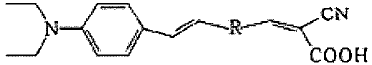

In the formula, R can be a substituted group having a thienothiophene structure.

Typically, a structural change in the amine unit as the electron donor brings about electronic property change, e.g. a light absorption spectrum shifted to blue color, and changes the length of the π-conjugation to adjust the light absorption spectrum and redox potential.

However, as most of organic dyes known provide lower conversion efficiency and driving stability than the ruthenium metal complexes do, there has been continuous efforts to develop a new dye that provides better molar extinction coefficient and higher photoelectric conversion efficiency than existing organic dye compounds do, by changing the type or the length of π-conjugation of the electron donor and electron acceptor.

### [Disclosure]

### [Technical Problem]

Accordingly, it is an aspect of the present invention to provide an organic dye which has a better molar extinction coefficient and higher photoelectric conversion efficiency than a conventional metal complex dye does to significantly enhance efficiency of a solar cell, and a preparation thereof.

Also, it is another aspect of the present invention to provide a dye-sensitized photoelectric conversion element which provides enhanced photoelectric conversion efficiency and good J_{sc} (short circuit photocurrent density) and molar extinction coefficient by including the dye, and a solar cell having better efficiency.

Additional aspects and/or advantages of the present invention will be set forth in part in the description which follows and, in part, will be obvious from the description, or may be learned by practice of the present invention.

### [Technical Solution]

The foregoing and/or other aspects of the present invention are achieved by providing a thienothiophene-based dye which is represented by following compounds 142 to 157:

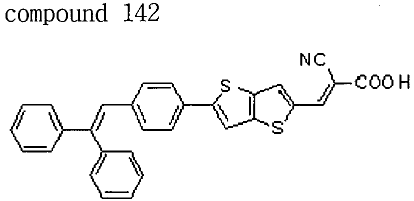

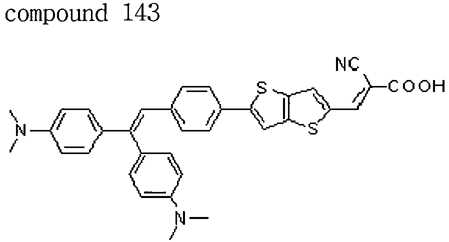

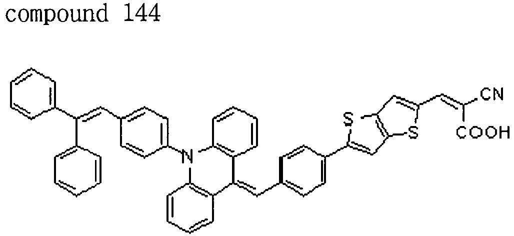

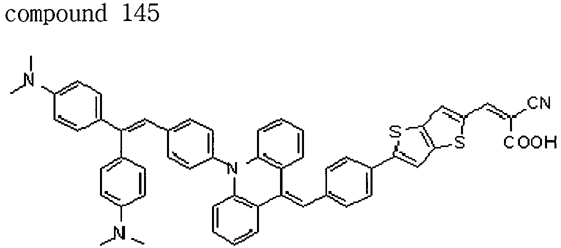

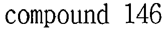

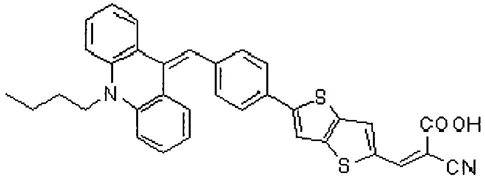

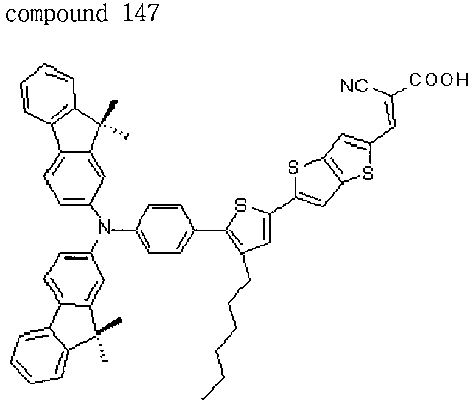

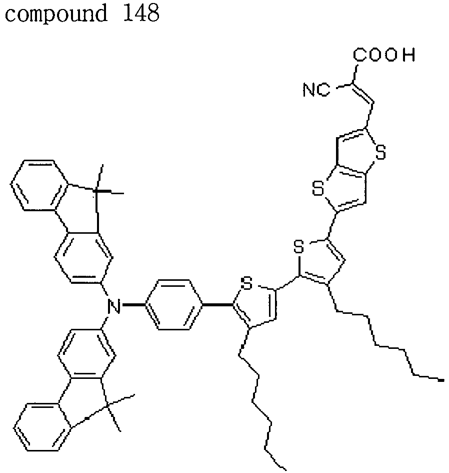

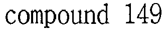

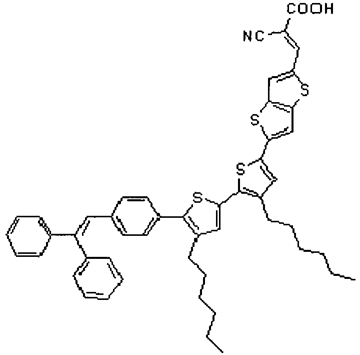

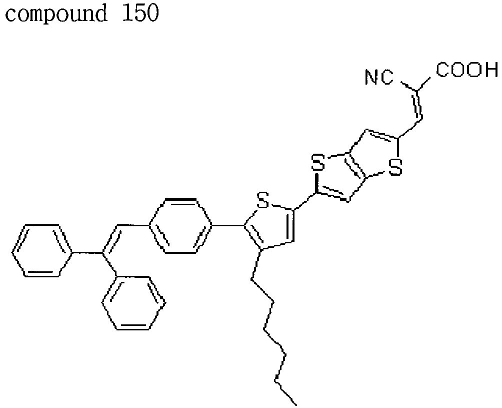

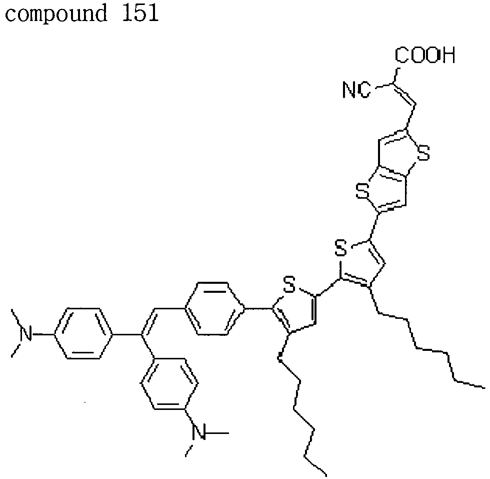

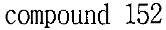

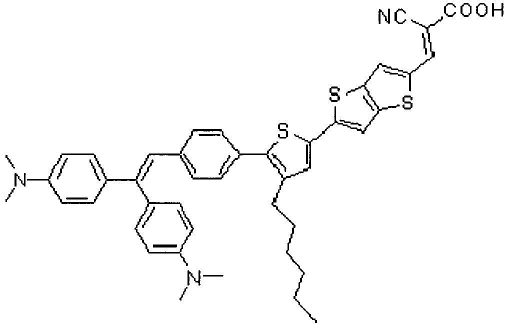

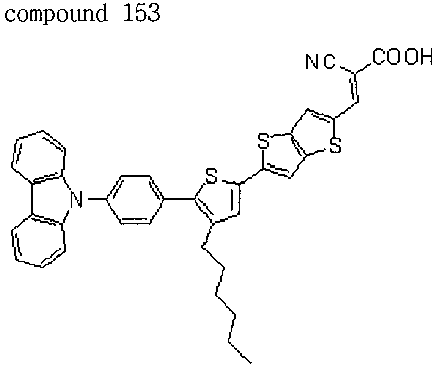

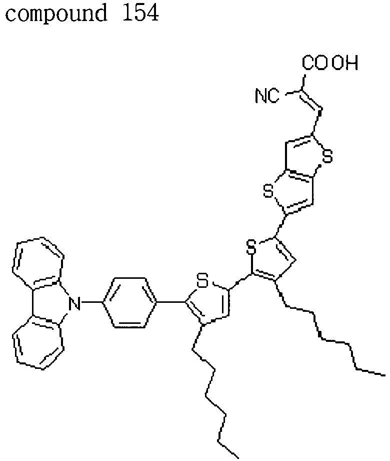

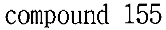

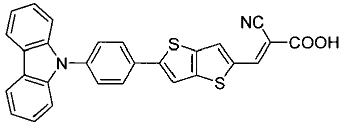

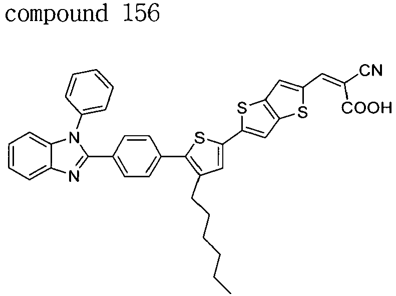

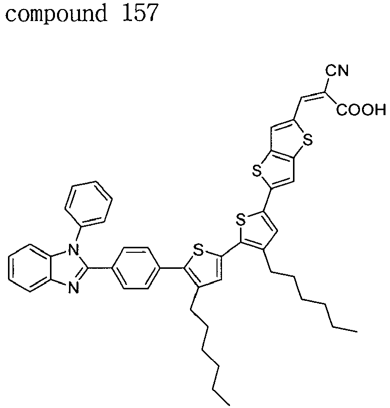

Compounds 142 to 157 are special embodiments of a thienothiophene-based dye which is represented by a chemical formula 4 below 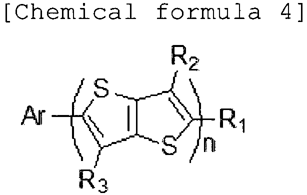 wherein, Ar comprises alkyl, aryl, alkoxy or heteroaryl of C₁₋₅₀ which is substituted or not substituted by alkyl, alkoxy, halogen, amide, cyano, hydroxyl, nitro, acyl, aryl or heteroaryl group of C₁₋₅₀;
R₁ is 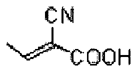 or 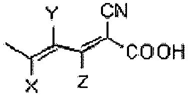 and X, Y and Z comprise hydrogen or alkyl, alkoxy or heteroalkyl of C₁₋₃₀;
R₂ and R₃ comprise hydrogen, halogen, amide, cyano, hydroxyl, nitro, acyl, C₁₋₃₀ alkyl, C₁₋₃₀ alkoxy, C₁₋₃₀ alkylcarbonyl or C₆₋₂₀ aryl, independently, and these are coupled with each other to form an oxygen-containing hetero ring if they are C₁₋₃₀ alkoxy; and
n is an integer number from 1 to 10.

The foregoing and/or other aspects of the present invention are also achieved by providing a preparation of a dye which is represented by a chemical formula 4, wherein, R₁ is 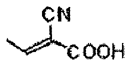 or 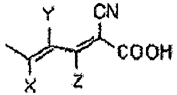 and X, Y and Z comprise hydrogen or alkyl, alkoxy or heteroalkyl of C₁₋₃₀;
R₂ and R₃ comprise hydrogen, halogen, amide, cyano, hydroxyl, nitro, acyl, C₁₋₃₀ alkyl, C₁₋₃₀ alkoxy, C₁₋₃₀ alkylcarbonyl or C₆₋₂₀ aryl, independently, and these are coupled with each other to form an oxygen-containing hetero ring if they are C₁₋₃₀ alkoxy; and
n is an integer number from 1 to 10; and
Ar is 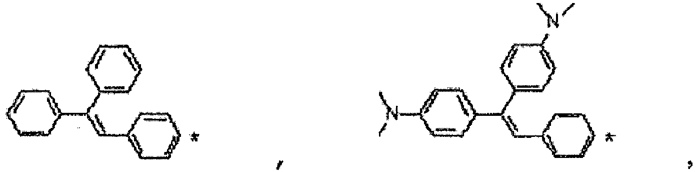 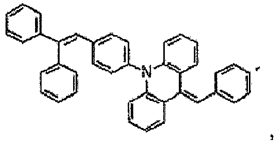 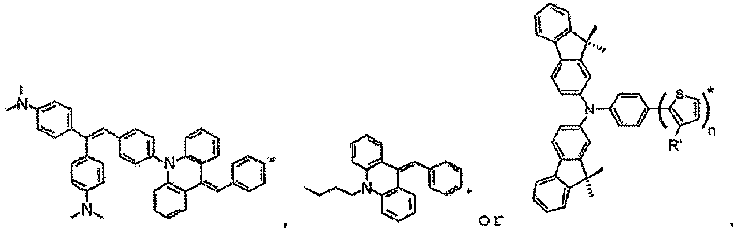 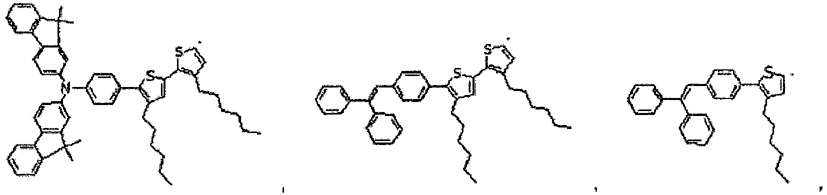 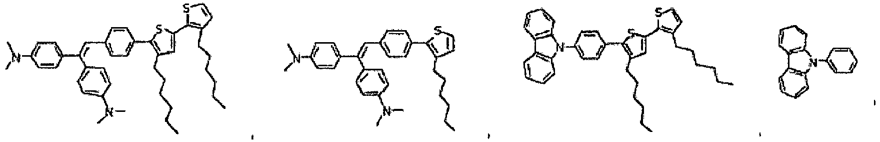 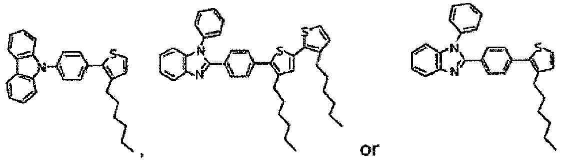 (here R' is alkyl having 1 to 6 carbon atoms, n is an integer number from 0 to 5 and * is a coupling)
independently, rings of aryl and heteroaryl groups have at least one substituent therein and the substituent may include an alkyl group, an alkoxy group, a halogen atom, an amide group, a cyano group, a hydroxyl group, a nitro group, an acyl group, an aryl group or heteroaryl of C₁₋₃₀, independently,
including a compound in a chemical formula 5 below reacting with n-butyllithium and then with a compound in a chemical formula 6 below

[Chemical formula 5] Ar-H₁

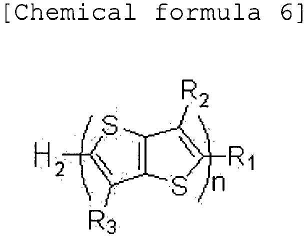

wherein R₁, R₂, R₃, Ar and n are as defined above, and H₁ and H₂ comprise halogen, independently.

The foregoing and/or other aspects of the present invention are also achieved by providing a dye-sensitized photoelectric conversion element which comprises oxide semiconductor particles applied with the compound represented by one of compounds 142 to 157 above.

The foregoing and/or other aspects of the present invention are also achieved by providing a dye-sensitized solar cell which comprises the dye-sensitized photoelectric conversion element.

### [Advantageous Effects]

A thiophene-based dye according to the present invention may greatly enhance efficiency of a solar cell by providing better molar extinction coefficient, J_{sc} (short-circuit photocurrent density) and photoelectric conversion efficiency than a conventional metal complex dye and drastically lower dye synthesis cost by being purified without expensive column.

### [Mode for Invention]

Hereinafter, exemplary embodiments of the present invention will be described with reference to accompanying drawings, wherein like numerals refer to like elements and repetitive descriptions will be avoided as necessary.

Hereinafter, the present invention will be described in detail.

The present inventors have discovered that a dye sensitized solar cell which is manufactured by applying a thiophene dye compound which is represented by one or more of compounds 142 to 157 above having a new organic dye structure, to oxide semiconductor particles, provides better efficiency with higher photoelectric conversion efficiency, Jsc (short circuit photocurrent density) and molar extinction coefficient than an existing dye-sensitized solar cell does, and have completed the present invention.

The present invention provides a thiophene-based dye represented by one of compounds 142 to 157 which are special embodiments of a following chemical formula 4.

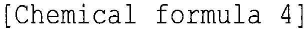

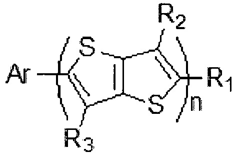

In the chemical formula 4, Ar includes alkyl, aryl, alkoxy or hetero aryl of C₁₋₅₀ which is substituted or not substituted by alkyl, alkoxy, halogen, amide, cyano, hydroxyl, nitro, acyl, aryl or heteroaryl group of C₁₋₅₀;
R₁ is 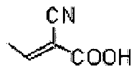 or 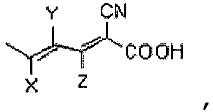 and X, Y and Z may be hydrogen or alkyl, alkoxy or heteroalkoxy of C₁₋₃₀, indenpendently;
R₂ and R₃ include hydrogen, halogen, amide, cyano, hydroxyl, nitro, acyl, C₁₋₃₀ alkyl or C₁₋₃₀ alkoxy, independently, which are coupled with each other to form an oxygen-containing hetero ring if they are C₁₋₃₀; and
n is a positive number from 1 to 10.
Ar can include 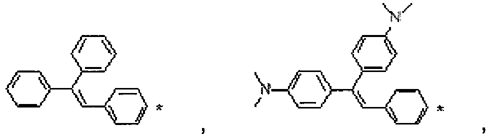 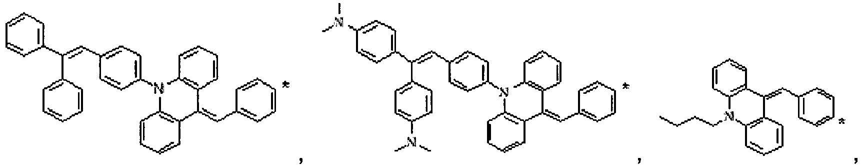 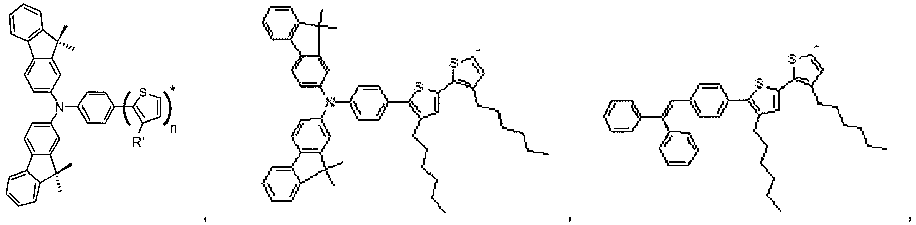 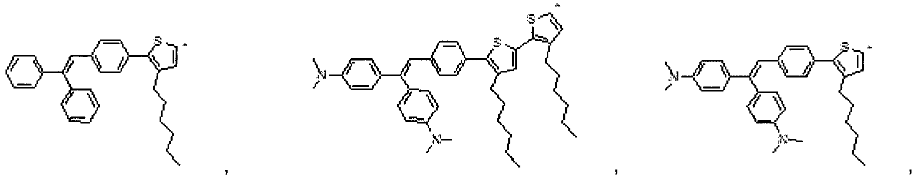 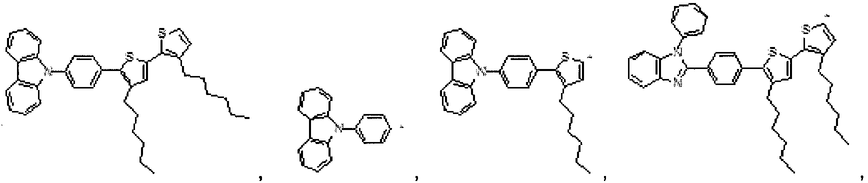 or 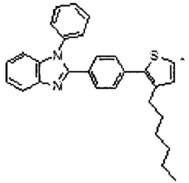 (here, R' is alkyl having 1 to 6 carbon atoms, n is a positive number from 0 to 5 and * is a coupling, independently), rings of aryl and heteroaryl groups may have at least one substituent therein, and the substituent may include alkyl group, alkoxy group, halogen atom, amide group, cyano group, hydroxyl group, nitro group, acyl group, aryl group or heterogryl of C₁₋₃₀, independently.

The present invention relates one of compounds which are represented by following compounds 142 to 157.

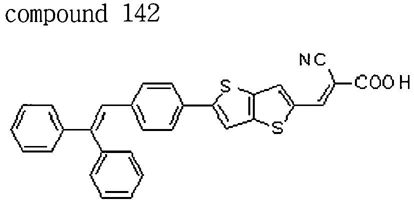

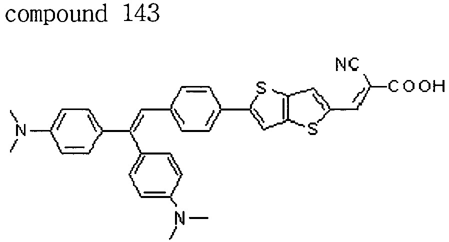

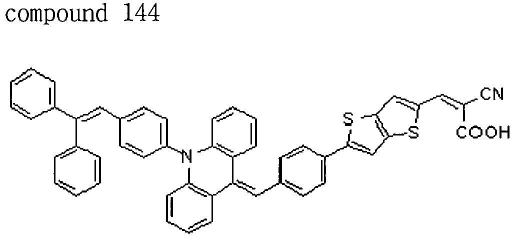

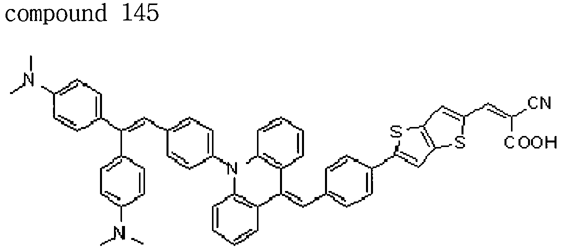

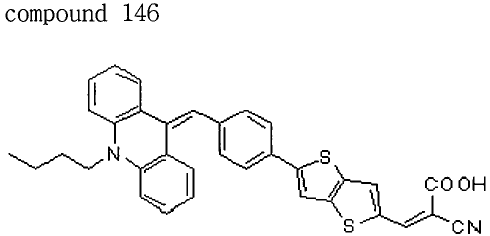

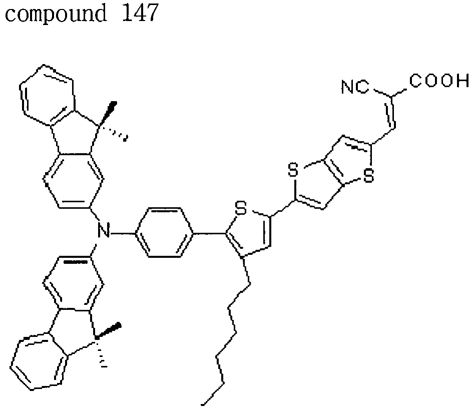

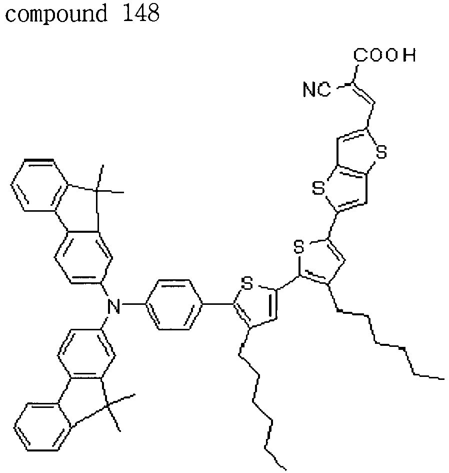

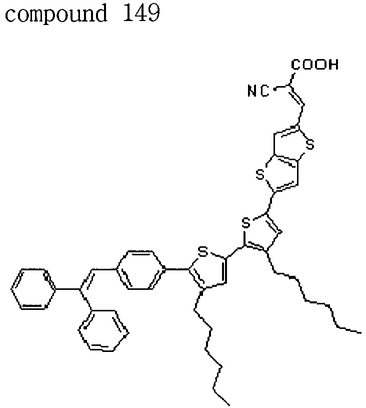

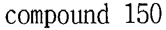

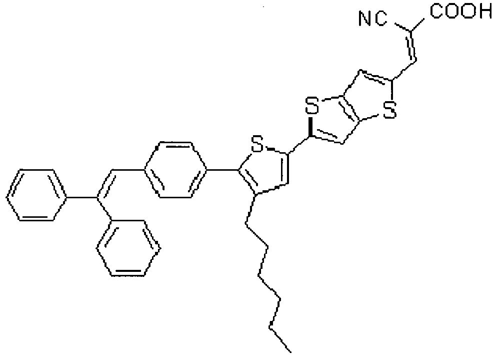

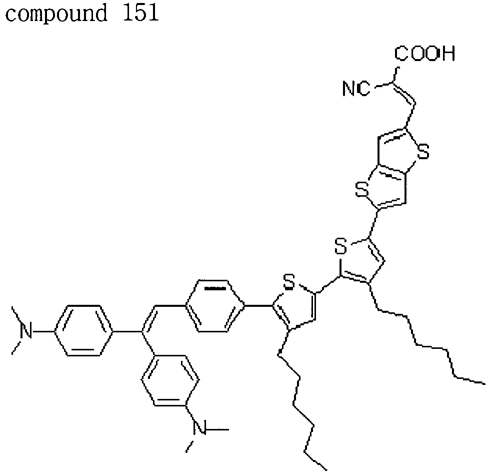

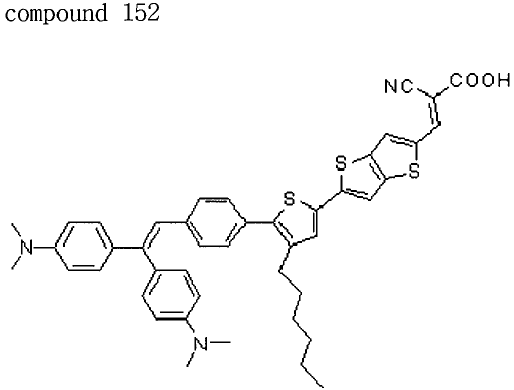

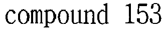

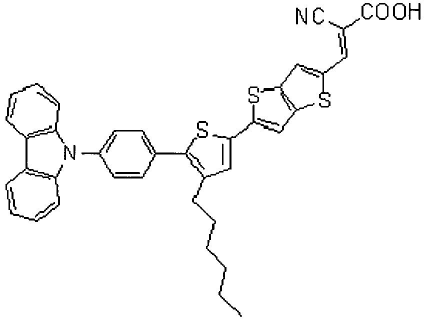

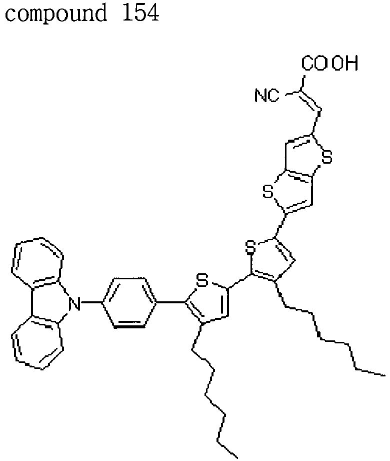

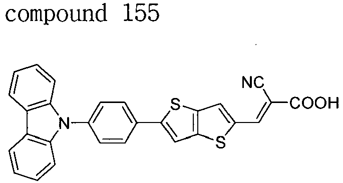

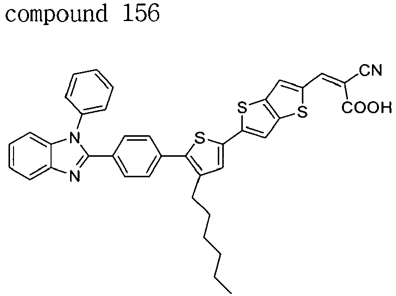

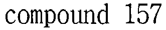

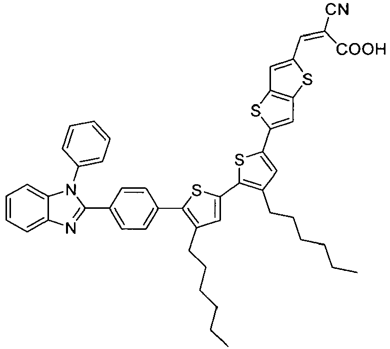

Further, the present invention provides a preparation of the dye represented by the chemical formula 4, wherein
R₁ is 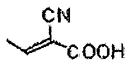 or 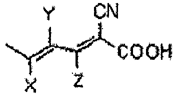 and X, Y and Z comprise hydrogen or alkyl, alkoxy or heteroalkyl of C₁₋₃₀;
R₂ and R₃ comprise hydrogen, halogen, amide, cyano, hydroxyl, nitro, acyl, C₁₋₃₀ alkyl, C₁₋₃₀ alkoxy, C₁₋₃₀ alkylcarbonyl or C₆₋₂₀ aryl, independently, and these are coupled with each other to form an oxygen-containing hetero ring if they are C₁₋₃₀ alkoxy; and
n is an integer number from 1 to 10; and
Ar is 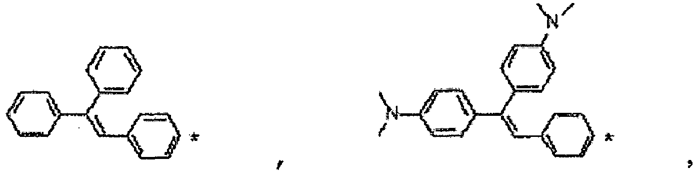 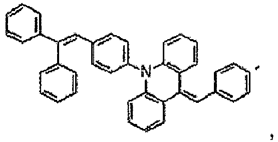 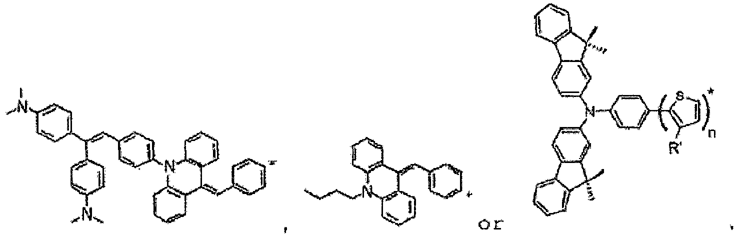 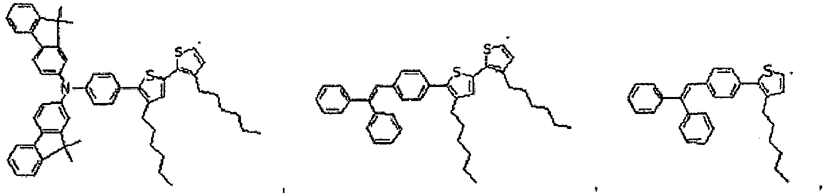 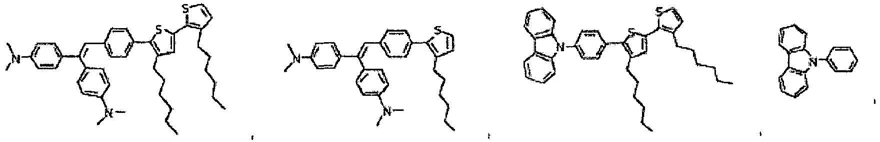 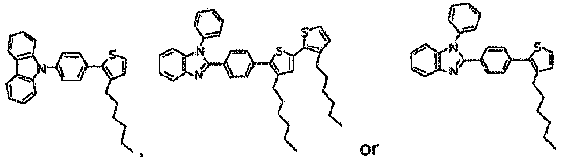 (here R' is alkyl having 1 to 6 carbon atoms, n is an integer number from 0 to 5 and * is a coupling)
independently, rings of aryl and heteroaryl groups have at least one substituent therein and the substituent may include an alkyl group, an alkoxy group, a halogen atom, an amide group, a cyano group, a hydroxyl group, a nitro group, an acyl group, an aryl group or heteroaryl of C₁₋₃₀, independently. The dye which is represented by the chemical formula 4 may be prepared by having a compound in a following chemical formula 5 react to n-butyllithium and then to a compound in a following chemical formula 6.

[Chemical formula 5] Ar-H₁

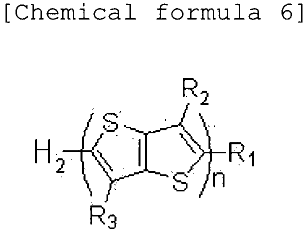

In the chemical formulas 5 and 6, R₁, R₂, R₃, Ar and n are as defined in the chemical formula 4.

H₁ and H₂ include halogen, independently.

More specifically, after melting the compound in the chemical formula 5 with an organic solvent (e.g. tetrahydrofurane (THF)), the melted compound may react to n-butyllithium at low temperatures and then with the compound in the chemical formula 6 at the same temperatures to obtain the desired compound in the chemical formula 4.

The compound in the chemical formula 5 which is used as a starting material in preparing the dye in the chemical formula 4 may be prepared by typical methods depending on the type of Ar.

Further, the present invention provides a dye-sensitized photoelectric conversion element. The dye-sensitized photoelectric conversion element includes oxide semiconductor particles carrying the dye represented by the compounds 142 to 157. The dye-sensitized photoelectric conversion element according to the present invention may be prepared with conventional dyes to be included in a solar cell, as well as prepared with the dye represented by the compounds 142 to 157. Preferably, the dye-sensitized photoelectric conversion element according to the present invention is prepared by forming an oxide semiconductor layer on a substrate with the oxide semiconductor particles and then by applying the dye according to the present invention to the thin film.

The substrate which has the oxide semiconductor particles layer according to the present invention preferably has a conductive surface. A commercial substrate may be used. More specifically, the substrate may include a glass or a transparent high molecular material such as polyethyleneterephthalate or polyethersulfone on which a conductive metal oxide layer such as tin oxide including indium, fluorine and antinomy or a metal layer such as steel, silver, gold and the like is formed. The conductivity is preferably 1000Ω and less, and more preferably, 100Ω and less.

The particles of the oxide semiconductor preferably include metal oxide. More specifically, the metal oxide may include titan, tin, zinc, tungsten, zirconium, gallium, indium, yttrium, niobium, tantalum, vanadium and the like. The metal oxide preferably includes titan, tin, zinc, niobium, indium and the like, and more preferably, titanium dioxide, zinc oxide and tin oxide and most preferably, titanium dioxide. The oxide semiconductor may be used alone, mixed with others or coated on a surface of a semiconductor.

A diameter of the oxide semiconductor particles is an average of 1 to 500nm, and more preferably, 1 to 100nm. The oxide semiconductor particles may include large and small diameters combined. Alternatively, the oxide semiconductor particles may include multiple layers.

The oxide semiconductor layer may be formed by spraying oxide semiconductor particles on a substrate, by electrically extracting a semiconductor particles layer from a substrate as an electrode or by applying a paste on a substrate to be dried, cured or fired. The paste includes particles which are created by hydrolyzing a precursor of semiconductor particles such as slurry of semiconductor particles or semiconductor alkoxide and the like. Preferably, the oxide semiconductor layer is formed by applying the paste on the substrate. In this case, the secondary-coagulated oxide semiconductor particles are dispersed in a dispersion medium by a known method to have an average primary diameter of 1 to 200nm to thereby form the slurry.

The dispersion medium which disperses the slurry may vary as long as it disperses the semiconductor particles. More specifically, the dispersion medium may include water, alcohol such as ethanol, ketone such as acetone, acetyl acetone or hydrocarbon such as hexane, which may be mixed and used. Preferably, the dispersion medium includes water since it less changes viscosity of the slurry. A dispersion stabilizer may be used to stabilize dispersion of the oxide semiconductor particles. More specifically, the dispersion stabilizer may include e.g., acid such as acetic acid, hydrochloric acid and nitric acid, acetyl acetone, acrylic acid, polyethyleneglycol, polyvinylalcohol and the like.

The substrate which is applied with the slurry may be fired at firing temperatures of 100°C and above, and preferably, 200°C and above. The upper limit of the firing temperatures is the melting point (softening point) of the material, i.e., 900°C, and preferably, 600°C and below. The firing temperatures according to the present invention are not limited particularly, but preferably within 4 hours.

According to the present invention, a thickness of the layer on the substrate may be 1 to 200µm, and preferably, 1 to 50µm. If the substrate is fired, some of the oxide semiconductor particles layer are melted and attached, but do not affect the present invention particularly.

The oxide semiconductor layer may be secondary treated. For example, the layer may be applied with a solution of alkoxide, chloride, nitride and sulfide which are the same metal as a semiconductor, and then dried or fired again to improve performance. The metal alkoxide may include titanethoxide, titaniumisoproepoxide, titan t-butoxide, n-dibutyldiacetyl tin and the like, or an alcohol solution thereof. The chloride may include e.g., titanium tetrachloride, tin tetrachloride, zinc chloride and the like, or an aqueous solution thereof. The obtained oxide semiconductor layer includes particles of the oxide semiconductor.

A method of applying the dye to the oxide semiconductor particles layer according to the present invention is not limited to a particular method. More specifically, the substrate having the oxide semiconductor layer may be dipped into a solution which is made by dissolving the dye represented by the compounds 142 to 157 with a solvent or dipped into a dispersion solution which is made by dispersing the dye. The concentration of the solution or the dispersion solution may be properly determined depending on the dye. The applying temperature ranges from normal temperatures to the boiling point of the solvent. The applying time ranges from one minute to 48 hours. More specifically, the solvent which dissolves the dye may include e.g., methanol, ethanol, acetonitrile, dimethylsulfoxide, dimethylformamide, acetone, t-butanol, etc. The dye concentration of the solution is typically 1X10⁻⁶M to 1M, and preferably, 1X10⁻⁵M to 1X10⁻¹M. Thus, the present invention may provide a dye-sensitized photoelectric conversion element which includes the oxide semiconductor particles layer.

The dye which is represented by one of compounds 142 to 157 may include a single or several dyes mixed. If the dyes are mixed, or other dyes or metal complex dyes may be mixed together. The metal complex dye to be mixed may include e.g., ruthenium complex or triarylmethylium salt thereof, phthalocyanine, porphyrin, etc., but not limited thereto. The organic dye to be mixed may include metal-free phthalocyanine, porphyrine, cyanine, merocyanine, oxonol, triphenylmethane, metin dye such as acrylic acid dye disclosed in WO2002/011213, xanthen dye, azo dye, anthraquinone dye, perylene dye and the like (refer to M. K. Nazeeruddin, A. Kay, I. Rodicio, R. Humphry-Baker, E. Müller, P. Liska, N. Vlachopoulos and M. Grätzel, J. Am. Chem. Soc., 1993, vol. 115, p. 6382). If at least two dyes are used, the dyes may sequentially be applied to the semiconductor layer, or mixed and melted together to be applied to the semiconductor layer.

If the oxide semiconductor particles layer according to the present invention is dipped into the dyes, the layer may be dipped into the dye under the presence of an inclusion compound to prevent the dyes from being bonded to one another. The inclusion compound may include cholic acid such as deoxycholic acid, dehydrodeoxycholic acid, chenodeoxycholic acid, cholic acid methylester, cholic acid natrium and the like, a steroid compound such as polethyleneoxide, cholic acid and the like, crown ether, cyclodextrin, calixarene, polyethyleneoxide, etc.

After being dipped into the dye, the electrode surface of the semiconductor layer may be treated by an amine compound such as 4-t-butyl pyridine or by a compound such as acetic acid, propionic acid which has an acidic group. For example, the substrate which has the semiconductor particles layer carrying the dye may be dipped into an ethanol solution of amine.

Further, the present invention provides a dye-sensitized solar cell which includes the dye-sensitized photoelectric conversion element. The solar cell may be manufactured by a known method by using a conventional photoelectric conversion element as well as using the dye-sensitized photoelectric conversion element having the oxide semiconductor particles layer carrying the dye represented by compounds 142 to 157. More specifically, the dye-sensitized solar cell may include an electrode (negative) of a photoelectric conversion element which is formed by applying the dye represented by the compounds 142 to 157 to the oxide semiconductor particles layer, a counter electrode (positive), a redox electrolyte, a hole transferring material or a p-type semiconductor.

Preferably, the preparation of the dye-sensitized solar cell according to the present invention includes an operation of coating TiO₂ paste on a transparent conductive substrate, an operation of firing the paste-coated substrate to form a TiO₂ layer, an operation of dipping the substrate having the TiO₂ layer into a solution including the dissolved dye represented by one or more of compounds 142 to 157 to form a TiO₂ film electrode having the dye, an operation of providing a second glass substrate including a counter electrode on the TiO₂ film electrode, an operation of forming a hole to pass through the second glass substrate and the counter electrode, an operation of coupling the counter electrode and the TiO₂ film electrode by heat and pressure, leaving a thermoplastic polymer film therebetween, an operation of injecting an electrolyte to the thermoplastic polymer film interposed between the counter electrode and the TiO₂ film electrode through the hole and an operation of sealing the thermoplastic polymer film.

The redox electrolyte, the hole transferring material and the p-type semiconductor may be liquid, coagulate (gel and gel phase), solid, etc. The redox electrolyte, the dissolved salt, the hole transferring material and the p-type semiconductor may be liquid by being dissolved by a solvent or may include normal temperature-molten salt. The redox electrolyte, the hole transferring material and the p-type semiconductor may be coagulate (gel and gel phase) by being included in a polymer matrix or a monomer gelation agent. The redox electrolyte, the dissolved salt, the hole transferring material and the p-type semiconductor may be solid.

The hole transferring material may include an amine derivative, a conductive polymer such as polyacetylene, polyaniline, polythiophene, a material such as a triphenylene compound using a discotheque liquid crystal phase, etc. The p-type semiconductor may include CuI, CuSCN and the like. Preferably, the counter electrode is conductive and acts as a catalyst for a reduction reaction of the redox electrolyte. For example, platinum, carbon, rhodium or ruthenium may be deposited on a glass or a polymer film, or conductive particles may be applied to the glass or the polymer film to form the counter electrode.

The redox electrolyte of the solar cell according to the present invention may include a halogen oxidation reduction electrolyte including a halogen compound having a halogen ion as a counter ion and a halogen molecule, a metal redox electrolyte such as ferrocyanide-ferrocyanide, ferrocene-ferricinium ion or a metal complex such as a cobalt complex, an organic oxidation reduction electrolyte such as alkylthiol-alkyldisulfide, viologen dye and hydroquinone-quinone and the like. Preferably, the redox electrolyte includes a halogen oxidation reduction electrolyte. Preferably, the halogen molecule of the halogen oxidation reduction electrolyte including the halogen compound-halogen molecule includes an iodine molecule. The halogen compound which has a halogen ion as a counter ion may include halogenated metal salt such as LiI, NaI, KI, CaI₂, MgI₂, CuI, halogen organic ammonium salt such as tetraalkylammoniumiodine, imidazoliumiodine, phyridiumiodine, or I₂.

The redox electrolyte may include a solution added with the foregoing materials. In this case, a solvent may electrochemically be inactive. More specifically, the solvent may include e.g., acetonitrile, propylenecarbonate, ethylenecarbonate, 3-methoxypropionitrile, methoxyacetonitrile, ethyleneglycol, propyleneglycol, diethyleneglycol, triethyleneglycol, butylolactone, dimethoxyethane, dimethylcarbonate, 1,3-dioxolane, methylformate, 2-methyltetrahydrofurane, 3-methoxy-oxazolidine-2-on, sulforane, tetrahydrofurane, water and the like. Preferably, the solvent includes acetonitrile, propylenecarbonate, ethylenecarbonate, 3-methoxypropionitrile, ethyleneglycol, 3-methoxy-oxazolidine-2-on, butylolactone, etc. The solvent may be used alone or mixed together. The gel phase positive electrolyte may be formed by adding an electrolyte or an electrolyte solution to a matrix such as oligomer and polymer or by adding an electrolyte or an electrolyte solution to a monomer gelation agent. The concentration of the redox electrolyte may be 0.01 to 99wt%, and more preferably, 0.1 to 30wt%.

The solar cell according to the present invention may be manufactured by providing the photoelectric conversion element (negative) having the oxide semiconductor particles carrying the dye and the counter electrode (positive) facing the photoelectric conversion element and by injecting the solution including the redox electrolyte therebetween.

Hereinafter, exemplary embodiments of the present invention are provided to help understand the present invention. However, the present invention is not limited to following exemplary embodiments.

### [Exemplary embodiments]

### Synthesis of dye

All reactions were implemented under argon condition, and all solvents used were distilled by a proper reagent from Sigma-Aldrich. 1H NMR spectrum was measured by Varian Mercury 300 spectrometer. Absorbance and emission spectra were measured by Perkin-Elmer Lambda 2S UV-visible spectrophotometer and Perkin LS fluorescence spectrometer, respectively.

[Exemplary embodiment 1] Synthesis of compound 142

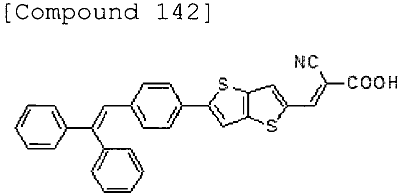

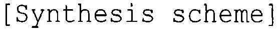

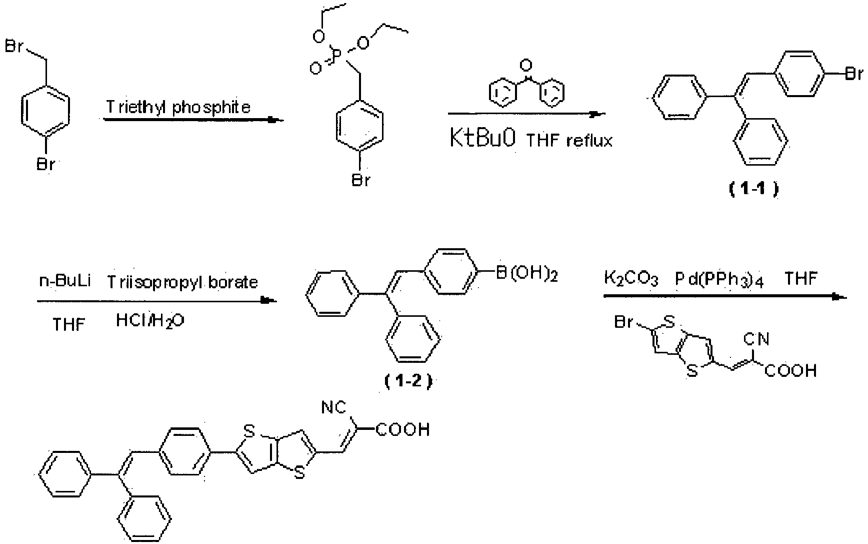

Together with triethylphosphite (25g, 150mmol), 1-bromo-4-bromomethylbenzene (5g, 20mmol) was put into an agitator to have them react to each other for two hours at 120°C to thereby synthesize triethyl 4-bromobenzylphosphite. The synthesized triethyl 4-bromobenzylphosphite (6.74g, 20mmol), benzophenone (5.13g, 29.16mmol) and potassium tert-butoxide (3.27g, 29.16mmol) were put into a reactor and melted by THF of 50ml to have them react to each other for four hours at 120°C to thereby synthesize 1-bromo-4-(2,2-diphenylvinyl)benzene(1-1). The synthesized-bromo-4-(2,2-diphenylvinyl)benzene(1-1) (6.03g, 18mmol) was melted by THF of 50ml, gradually added with 2Mn-BuLi (10ml, 20mmol) at -78°C and agitated for one hour at low temperatures. The mixture was gradually added with triisopropyl borate (3.76g, 20mmol) at -78°C, agitated for one hour at low temperatures and then additionally agitated for 30 minutes at 0°C to thereby synthesize 4-(2,2-diphenylvinyl)phenylboronic acid (1-2). The synthesized 4-(2,2-diphenylvinyl)phenylboronic acid (4.5g, 15mmol) (1-2), K₂CO₃ (6.2g, 45mmol) and Pd(PPh₃)₄(0.86g, 0.75mmol) were melted by THF (60ml), added with (E)-3-(5-bromothieno[3,2-b]thiophene-2-yl)-2-cyanoacrylic acid (5.6g, 18mmol), agitated for four hours at 120°C to complete the reaction. After extracting an organic layer from the mixture with dichloromethane and distilled water, the solvent was removed through an evaporator and the organic layer was recrystallized by n-hexane and dried to collect the compound 142 after the sediment was filtered.

[Exemplary embodiment 2] Synthesis of compound 144

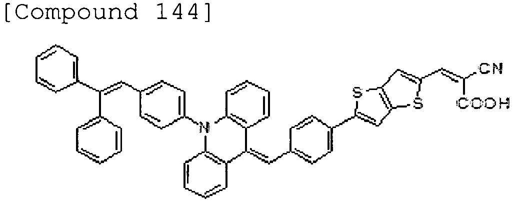

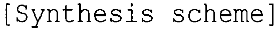

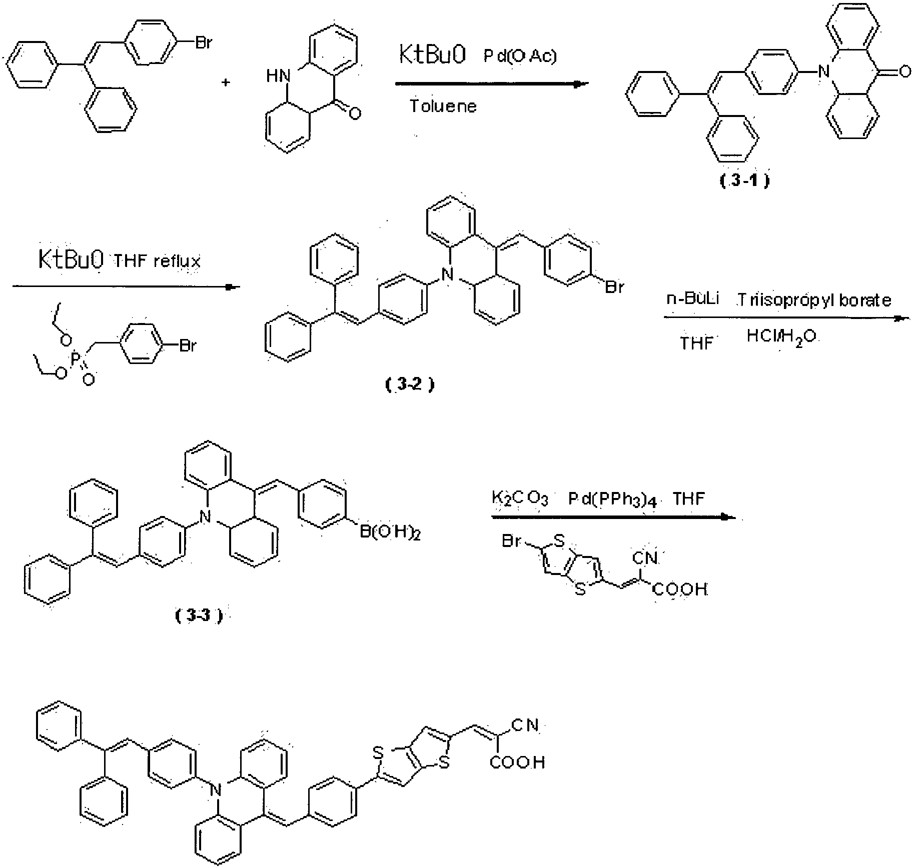

1-bromo-4-(2,2-diphenylvinyl)benzene (6.03g, 18mmol) and 10,,10,,-dihydroacridin-9(8aH)-one(4.26g, 21.6mmol) were put into a reaction container, added with KtBuO(4.84g, 43.2mmol) and Pd(OAc) (0.24g, 1.08mmol) and melted by toluene (50ml) to have them react to each other for 12 hours at 150°C to thereby synthesize a compound 10-(4-(2,2-diphenylvinyl)phenyl)-10,,10,,-dihydroacridin-9(8aH)-one(3-1). The synthesized compound 10-(4-(2,2-diphenylvinyl)phenyl)-10,,10,,-dihydroacridin-9(8aH)-one(3-1)(3.2g, 7.08mmol) and triethyl 4-bromobenzylphosphite (2.6g, 8.49mmol) were put into a reactor, added with KtBuO (0.95g, 8.49mmol), melted by THF (50ml) and agitated for four hours at 120°C to synthesize a compound (E)-9-(4-bromobenzylidene)-10-(4-(2,2-diphenylvinyl)phenyl)-8a,9,10,,10,,-tetrahydroacridine(3-2). The synthesized compound (E)-9-(4-bromobenzylidene)-10-(4-(2,2-diphenylvinyl)phenyl)-8a,9,10,,10,,-tetrahydroacridine(3-2) (3.5g, 5.78mmol) was melted by THF of 30ml, gradually added with 2Mn-BuLi (3.45g, 6.9mmol) at -78°C and agitated for one hour at low temperatures. The mixture was gradually added with triisoprophyl borate (1.29g, 6.9mmol) at -78°C, agitated for one hour at low temperatures and then additionally agitated for 30 minutes at 0°C to thereby synthesize a compound (E)-4-((10-(4-(2,2-diphenylvinyl)phenyl)-10,,10,,-dihydroacridin-9(8aH)-ylidene)methyl)phenylboronic acid (3-3). The synthesized compound (E)-4-((10-(4-(2,2-diphenylvinyl)phenyl)-10,,10,,-dihydroacridin-9(8aH)-ylidene)methyl)phenylboronic acid (3-3) (2.8g, 4.9mmol), K₂CO₃(2.03g, 14.7mmol) and Pd(PPh₃)₄(0.86g, 0.29mmol) were melted by THF (30ml), added with (E)-3-(5-bromothieno[3,2-b]thiophene-2-yl)-2-cyanoacrylic acid (5.6g, 18mmol) and agitated for four hours at 120°C to complete the reaction. After extracting an organic layer from the mixture with dichloromethane and distilled water, the solvent was removed through an evaporator and the organic layer was recrystallized by n-hexane, and dried to collect the compound 144 after the sediment was filtered.

### [Exemplary embodiment 3] Synthesis of compound 146

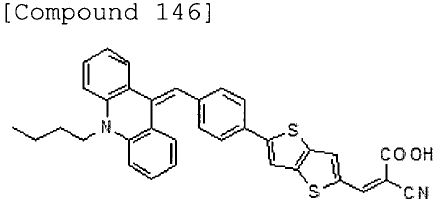

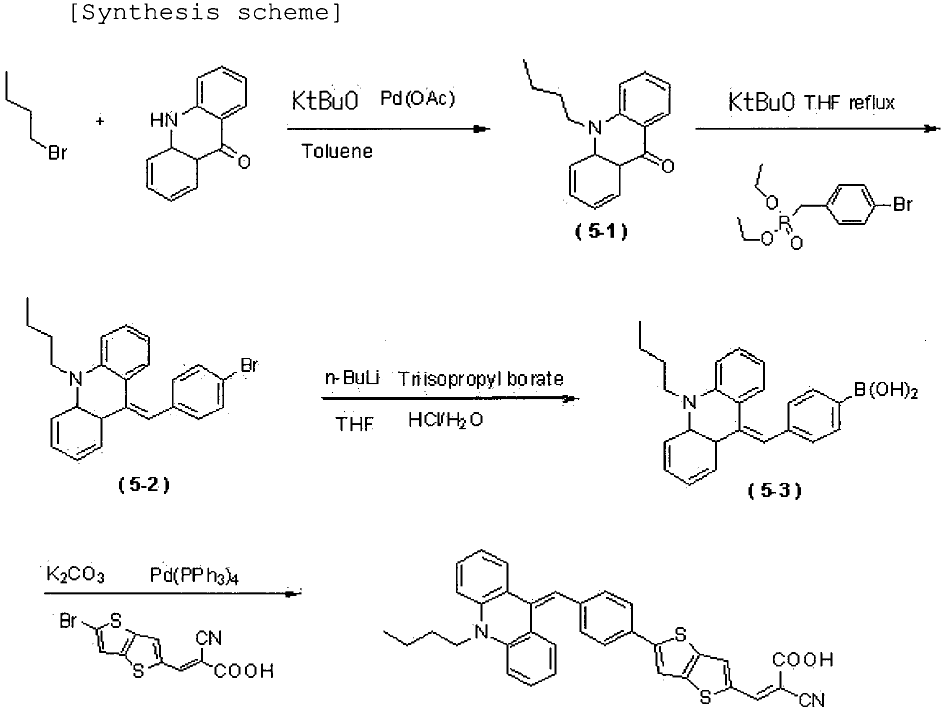

1-bromobutane (5g, 36.5mmol) and 10,,10,, - dihydroacridin-9(8aH)-one(7.19g, 36.5mmol) were put into a reactor, added with KtBuO(9.8g, 87.6mmol) and Pd(OAc)(0.49g, 2.19mmol) and melted by toluene (60ml) to have them react to one another for 12 hours at 150°C to thereby synthesize a compound 10-butyl-10,,10,, - dihydroacridin-9(8aH)-one(5-1). The synthesized compound 10-butyl-10,,10,,-dihydroacridin-9(8aH)-one(5-1) (4.2g, 16.5mmol) and triethyl 4-bromobenzylphosphite (6.08g, 19.8mmol) were put into a reactor, added with KtBuO (2.22g, 19.8mmol), melted by THF (50ml) and agitated for four hours at 120°C to thereby synthesize a compound (Z)-9-(4-bromobenzylidene)-10-butyl-8a,9,10,,10,,-tetrahydroacridine(5-2). The synthesized compound (Z)-9-(4-bromobenzylidene)-10-butyl-8a,9,10,,10,,-tetrahydroacridine(5-2) (6.09g, 15mmol) was melted by THF of 50ml, gradually added with Mn-BuLi (9ml, 18mmol) at - 78°C and agitated for one hour at low temperatures. The mixture was gradually added with triisopropyl borate (3.38g, 18mmol) at -78°C, agitated for one hour at low temperatures and then additionally agitated for 30 minutes at 0°C to thereby synthesize a compound (Z)-4-((10-butyl-10,,10,,dihydroacridin-9(8aH)-ylidene)methyl)phenylboronic acid (5-3). The synthesized compound (Z)-4-((10-butyl-10,,10,,dihydroacridin-9(8aH)-ylidene)methyl)phenylboronic acid (5-3)(4.82g, 13mmol), K₂CO₃(5.39g, 39mmol) and Pd(PPh₃)₄(0.99g, 0.65mmol) were melted by THF (30ml), added with (E)-3-(5-bromothieno[3,2-b]thiophene-2-yl)-2-cyanoacrylic acid (4.08g, 13mmol) and agitated for four hours at 120°C to complete the reaction. After extracting an organic layer from the mixture with dichloromethane and distilled water, the solvent was removed through an evaporator and the organic layer was recrystallized by n-hexane and dried to collect the compound 146 after the sediment was filtered.

[Exemplary embodiment 4] Synthesis of compound 147

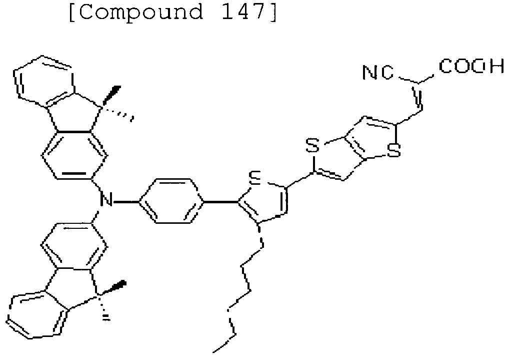

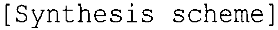

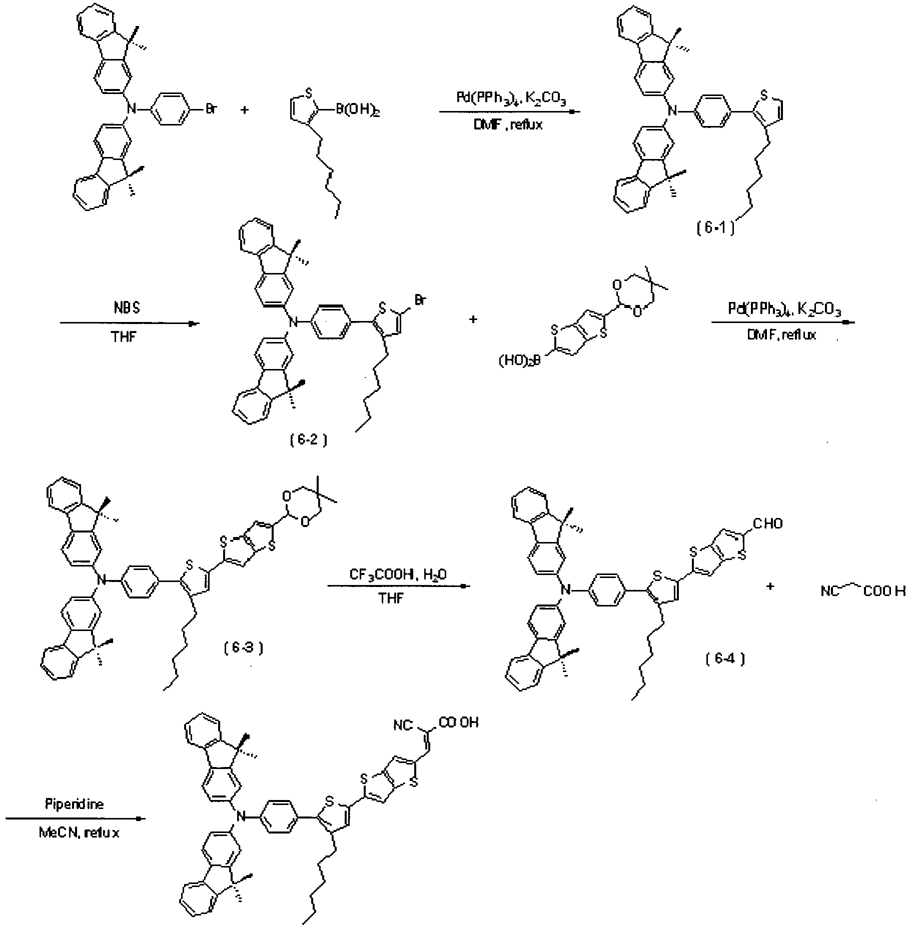

(4-bromo-phenyl)-bis-(9,9-dimethyl-9H-fluoren-2-yl)-amine (0.98g, 1.76mmol), 3-hexylthiophene-2-boronic acid (0.37g, 1.76mmol), tetrakis-(triphenylphosphine)palladium (0) (0.1g, 0.088mmol) and potassium carbonate (0.73g, 5.28mmol) were mixed, melted by DMF (40ml) and refluxed and agitated for 12 hours under nitrogen gas.

After the agitation, an organic layer was extracted from the mixture by using methylenechloride and water, evaporated and went through a column chromatography to synthesize N-(9,9-dimethyl-9H-fluoren-2-yl)-N-(4-(3-hexylthiophene-2-yl)phenyl)-9,9-dim-ethyl-9H-fluoren-2-amine **(6-1)**. (eluent. M.C : Hx = 1:5) 1H NMR(CDCl₃):[ppm] = 0.89(m, 3H), 1.29(m, 4H), 1.52(m, 4H), 2.52(m, 2H), 1.47 (s, 12H), 6.7 (d, ³J_{HH} = 2.4Hz, 1H), 6.91(d, ³J_{HH} = 2.4Hz, 1H), 7.09(m, 4H), 7.31(m, 4H), 7.38(m, 4H), 7.58(d, ³J_{HH} = 11.6Hz, 2H), 7. 62 (m, 4H).

The synthesized N-(9,9-dimethyl-9H-fluoren-2-yl)-N-(4-(3-hexylthiophene-2-yl)phenyl)-9,9-dim-ethyl-9H-fluoren-2-amine (6-1) (0.91g, 1.42mmol) and NBS(0.3g, 1.7mmol) were melted by THF (30ml) and agitated for four hours under nitrogen atmosphere.

After the agitation, an organic layer was extracted from the mixture by using methylenechloride and water, evaporated and went through a column chromatography to synthesize N-(4-(5-bromo-3-hexylthiophene-2-yl)phenyl)-N-(9,9-dimethyl-9H-fluoren-2-yl)-9,9-dimethyl-9H-fluoren-2-amine (6-2). (eluent. M.C : Hx = 1:5).

The synthesized N-(4-(5-bromo-3-hexylthiophene-2-yl)phenyl)-N-(9,9-dimethyl-9H-fluoren-2-yl)-9,9-dimethyl-9H-fluoren-2-amine **(6-2)** (1.01g, 1.4mmol), 5-(5,5-dimethyl-1,3-dioxan-2-yl)thieno[3,2-b]thiophene-2-ylboronic acid (0.42g, 1.4mmol), tetrakis-(triphenylphos-phine)palladium(0) (0.08g, 0.07mmol) and potassium carbonate (0.59g, 4.26mmol) were melted by THF (40ml) and refluxed and agitated for 12 hours under nitrogen gas.

After the agitation, an organic layer was extracted from the mixture by using methylenechloride and water, evaporated and went through a column chromatography to synthesize N-(4-(5-(5-(5,5-dimethyl-1,3-dioxan-2-yl)thieno[3,2-b]thiophene-2-yl)-3-hexy-lthiophene-2-yl)phenyl)-N-(9,9-dimethyl-9H-fluoren-2-yl)-9,9-dimethyl-9H-fluoren-2-amine **(6-3)**. (eluent. M.C : Hx = 1:4).

The synthesized N-(4-(5-(5-(5,5-dimethyl-1,3-dioxan-2-yl)thieno[3,2-b]thiophene-2-yl)-3-hexy-lthiophene-2-yl)phenyl)-N-(9,9-dimethyl-9H-fluoren-2-yl)-9,9-dimethyl-9H-fluoren-2-amine **(6-3)** (1.07g, 1.2mol) was melted by THF (30ml), added with trifluoroacetic acid (0.26ml) and water (1ml) and agitated for four hours under nitrogen atmosphere.

After the agitation, an organic layer was extracted from the mixture by using methylenechloride and water, evaporated and went through a column chromatography to synthesize 5-(5-(4-(bis(9,9-dimethyl-9H-fluoren-2-yl)amino)phenyl)-4-hexylthiophene-2-yl)-thieno[3,2-b]thiophene-2-carbaldehyde(6-4). (eluent. M.C:Hx = 1:1).

The synthesized 5-(5-(4-(bis(9,9-dimethyl-9H-fluoren-2-yl)amino)phenyl)-4-hexylthiophene-2-yl)-thieno[3,2-b]thiophene-2-carbaldehyde(6-4)(0.89g, 1.0mmol), cyanoacetic acid (0.1g, 1.2mmol) and piperidine (0.12ml, 1.2mmol) were melted by acetonitrile (30ml) and refluxed and agitated for four hours.

After the agitation, an organic layer was extracted from the mixture by using methylenechloride and water, evaporated and went through a column chromatography to synthesize a compound 147. (eluent. EA:MeOH = 10:1)

[Exemplary embodiment 5] Synthesis of Compound 148

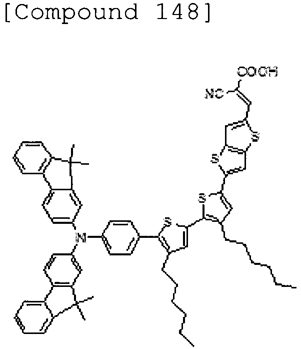

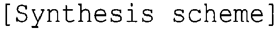

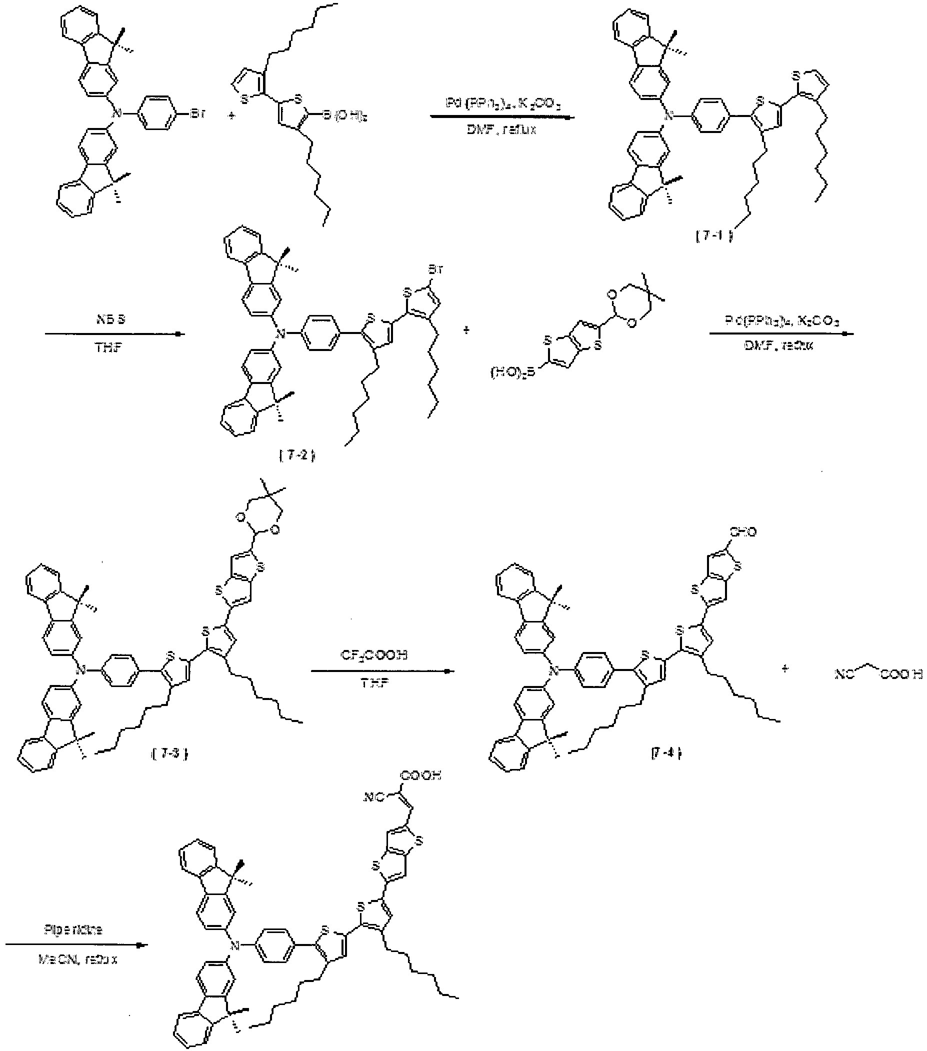

(4-bromo-phenyl)-bis-(9,9-dimethyl-9H-fluoren-2-yl)-amine (0.98g, 1.76mmol), 3',4-dihexyl-2,2'-bithiophene-5-ylboronic acid (0.66g, 1.76mmol), tetrakis-(tripheneylphos-phine)palladium(0) (0.1g, 0.088mmol) and potassium carbonate (0.73g, 5.28mmol) were melted by DMF (40ml) to synthesize a compound 148 with the same method as that in the exemplary embodiment 4.

[Exemplary embodiment 6] Synthesis of a compound of chemical formula 149

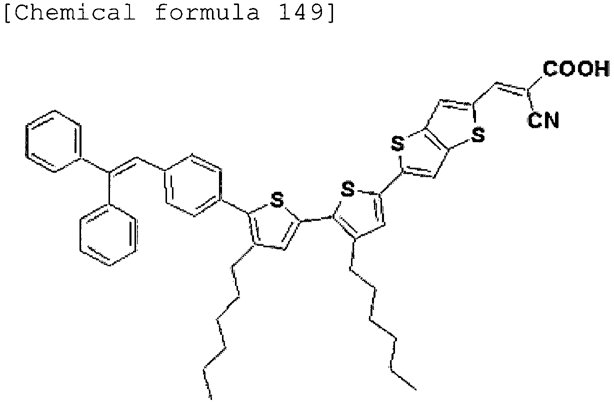

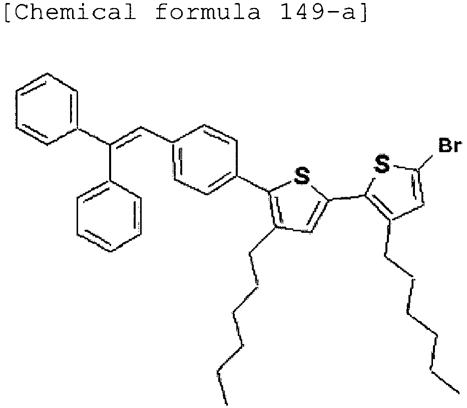

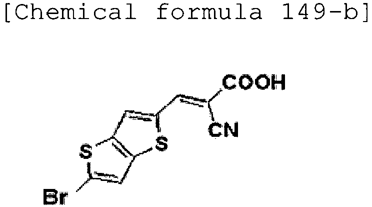

A compound in the chemical formula 149-a was melted by tetrahydrofurane (THF) and reacted to n-butyllithium and trialkyl borate consecutively at 0 to -100°C. The obtained reaction product reacted to a compound in the chemical formula 149-b in THF at 100 to 150°C to generate a compound in the chemical formula 149.

[Exemplary embodiment 7] Synthesis of a compound of chemical formula 150

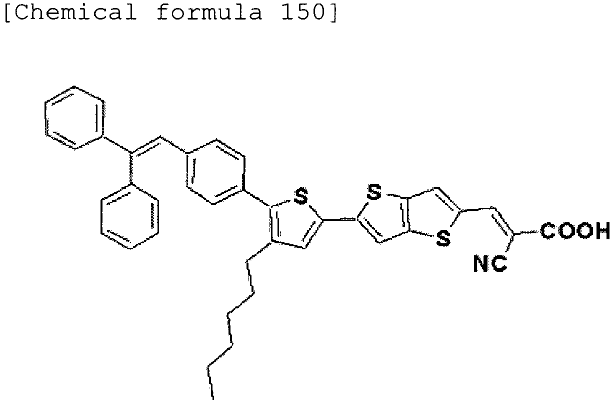

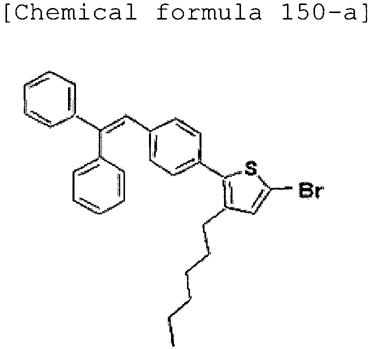

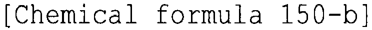

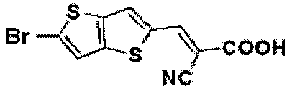

A compound in the chemical formula 150-a was melted by tetrahydrofurane (THF) and reacted to n-butyllithium and trialkyl borate consecutively at 0 to -100°C. The obtained reaction product reacted to a compound in the chemical formula 150-b in THF at 100 to 150°C to generate a compound in the chemical formula 150.

[Exemplary embodiment 8] Synthesis of compound 154

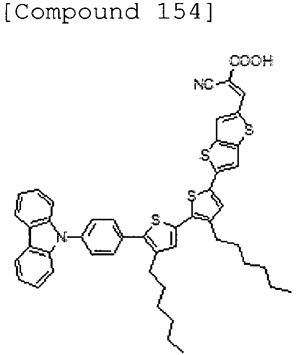

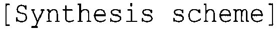

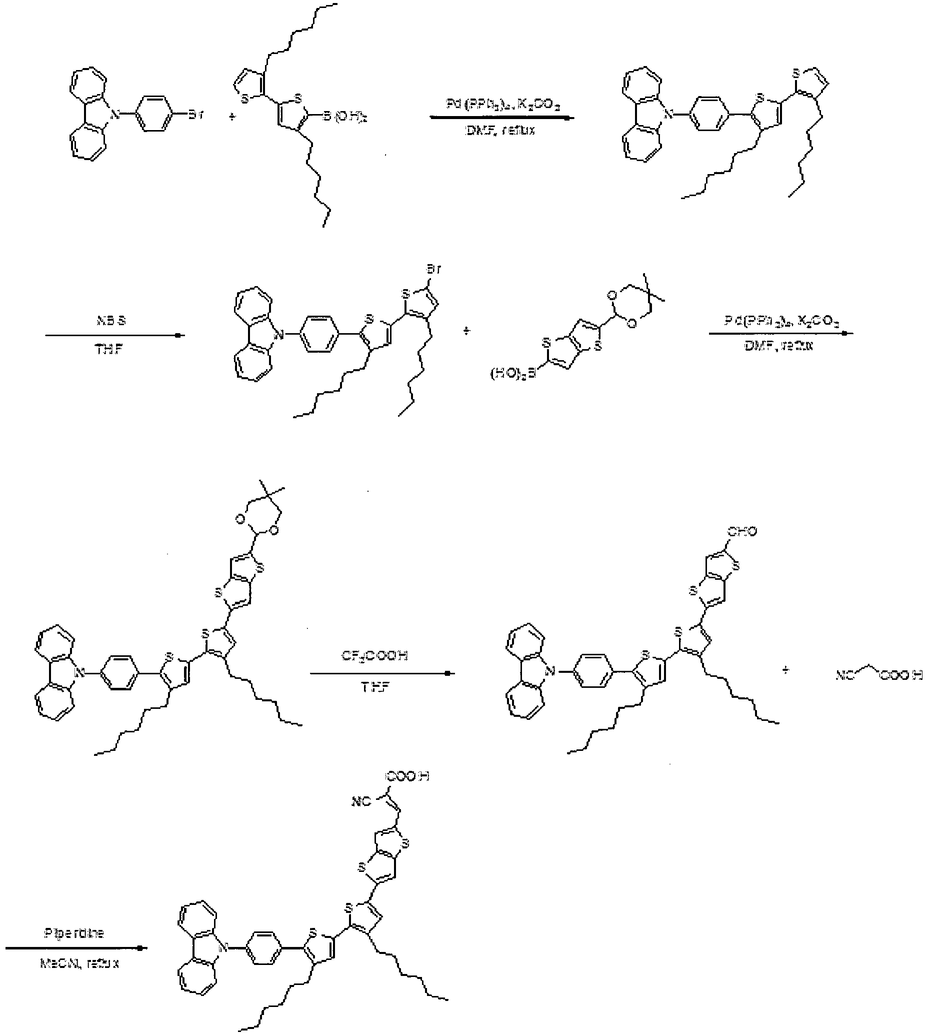

9-(4-bromophenyl)-9H-carbazole (0.56g, 1.76mmol), 3',4-dihexyl-2,2'-bithiophene-5-ylboronic acid (0.66g, 1.76mmol), tetrakis-(tripheneylphos-phine)palladium(0) (0.1g, 0.088mmol) and potassium carbonate (0.73g, 5.28mmol) were melted by DMF (40ml) to synthesize a compound 154 with the same method as that in the exemplary embodiment 4.

[Exemplary embodiment 9] Synthesis of compound 157

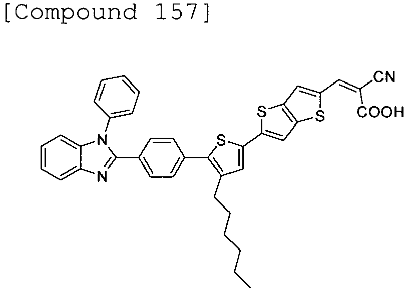

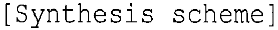

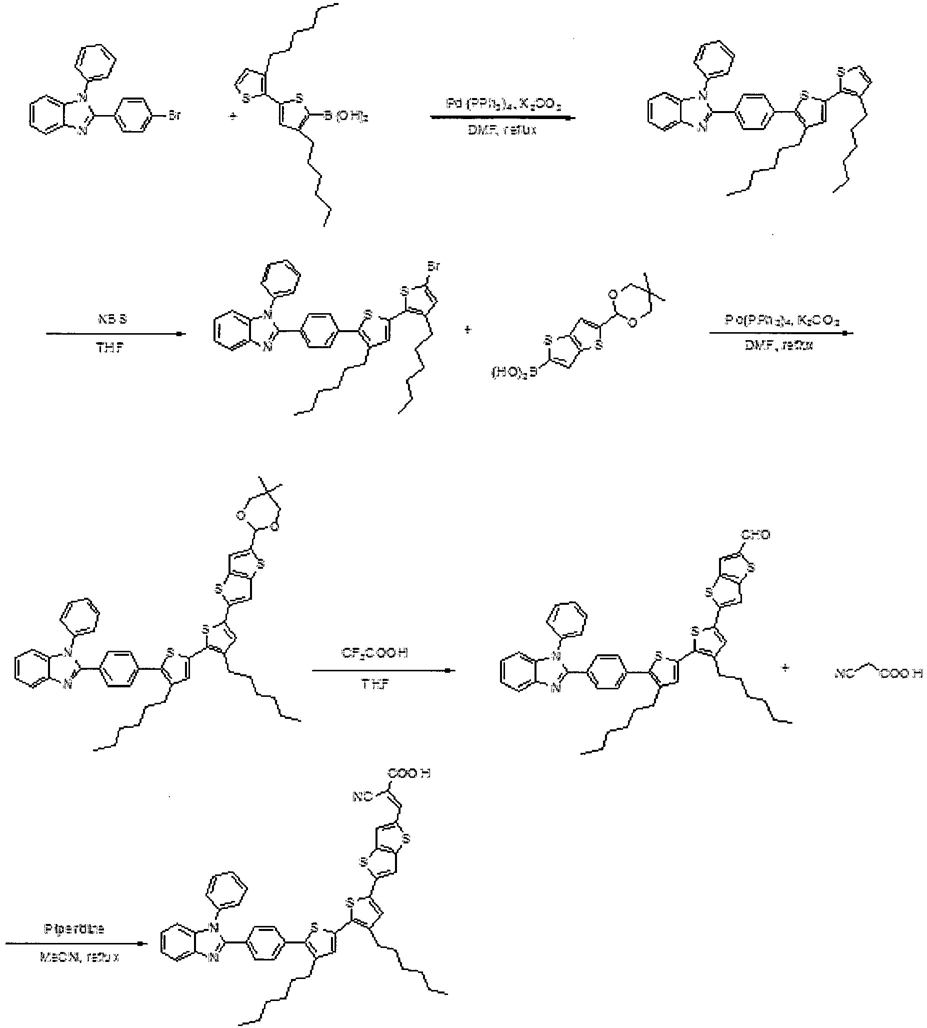

2-(4-bromophenyl)-1-phenyl-1H-benzo[d]imidazole (0.62g, 1.76mmol), 3',4-dihexyl-2,2'-bithiophen-5-ylboronic acid (0.66g, 1.76mmol), tetrakis-(tripheneylphos-phine)palladium(0) (0.1g, 0.088mmol) and potassium carbonate (0.73g, 5.28mmol) were melted by DMF (40ml) to synthesize a compound 157 with the same method as that in the exemplary embodiment 4.

### [Exemplary embodiment 10] Fabrication of dye-sensitized solar cell

A solar cell was manufactured by using a 12+8µm TiO₂ transparent layer to evaluate current-voltage properties of the dye compounds according to the present invention. A TiO₂ paste (solaronix, 13nm paste) was screen-printed to make a first TiO₂ layer in 12µm, and a second TiO₂ dispersion layer having a thickness of 8µm was made by using another paste (CCIC, HWP-400) for light dispersion. The TiO₂ double layer was treated by 40mN TiCl₄ liquid and dried for 30 minutes at 500°C. After cooling the treated film into 60°C, the film was applied with each solution of the dye compounds 142, 147, 148, 149, and 150(0.3mM dye of 10mM kenodioxycholic acid containing ethanol). The dye-absorbed TiO₂ electrode was coupled with a sandwich cell which was heated and sealed, leaving a high temperature molten film (Surlyn 1702, 25µm) as a spacer between platinum and counter electrode. The electrolyte liquid used was 0.6M 3-hexyl-1,2-dimethylimidazolium iodine, 0.04M I₂, 0.025M LiI, 0.05M guanidium thiocyanate and 0.28M *tert*-butylpiridine of acetonitrile.

### [Exemplary embodiment 11] Measurement of properties of dye and dye-sensitized solar cell prepared

The properties of the solar cell which was manufactured according to the exemplary embodiment 10 were measured and the result was shown in Table 1.

**[Table 1]**

| | Voc (V) | Isc (A) | Jsc (mA/ cm²) | Fill factor (%) | Test element dimension (cm²) | Efficiency (%) |
|---|---|---|---|---|---|---|
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |
| Compound 142 | 0.569 | 0.00150 | 6.065 | 72.36 | 0.248 | 2.50 |
| Compound 147 | 0.759 | 0.00260 | 10.08 | 59.6 | 0.258 | 4.46 |
| Compound 148 | 0.654 | 0.00162 | 14.96 | 76.4 | 0.116 | 6.97 |
| Compound 149 | 0.631 | 0.00144 | 10.19 | 75.0 | 0.141 | 4.82 |
| Compound 150 | 0.579 3 | 0.00264 | 9.982 | 70.86 | 0.264 | 4.10 |
| | | | | | | |
| | | | | | | |
| Ref. (N-719) | 0.775 | 0.00390 | 18.04 | 68 | 0.216 | 9.51 |

As seen from the result above, the dye according to the present invention provides good molar extinction coefficient, J_{sc} (short-circuit photocurrent density) and photoelectric conversion efficiency to drastically enhance efficiency of a solar cell.

Although a few exemplary embodiments of the present invention have been shown and described, it will be appreciated by those skilled in the art that changes may be made in these exemplary embodiments without departing from the principles and spirit of the invention, the scope of which is defined in the appended claims and their equivalents.

### [Industrial Applicability]

A thiophene-based dye according to the present invention may greatly enhance efficiency of a solar cell by providing better molar extinction coefficient, J_{sc} (short-circuit photocurrent density) and photoelectric conversion efficiency than a conventional metal complex dye and drastically lower dye synthesis cost by being purified without expensive column.

### [Items]

[Item 1]
   The present invention relates to special embodiments of a thienothiophene-based dye which is represented by a chemical formula 4 below: 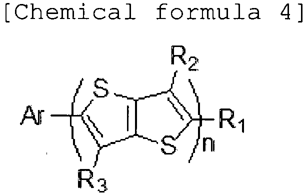 wherein, Ar comprises alkyl, aryl, alkoxy or heteroaryl of C₁₋₅₀ which is substituted or not substituted by alkyl, alkoxy, halogen, amide, cyano, hydroxyl, nitro, acyl, aryl or heteroaryl group of C₁₋₅₀;
   R₁ is 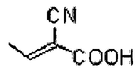 or 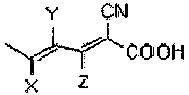 and X, Y and Z comprise hydrogen or alkyl, alkoxy or heteroalkyl of C₁₋₃₀;
   R₂ and R₃ comprise hydrogen, halogen, amide, cyano, hydroxyl, nitro, acyl, C₁₋₃₀ alkyl, C₁₋₃₀ alkoxy, C₁₋₃₀ alkylcarbonyl or C₆₋₂₀ aryl, independently, and these are coupled with each other to form an oxygen-containing hetero ring if they are C₁₋₃₀ alkoxy; and
   n is an integer number from 1 to 10.
[Item 2]
   The thienothiophene-based dye according to item 1, wherein Ar is 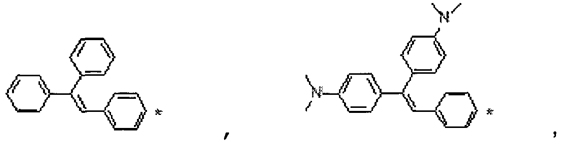 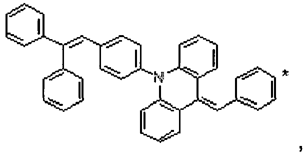 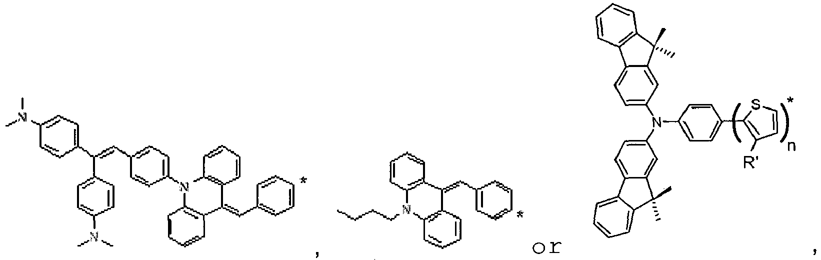 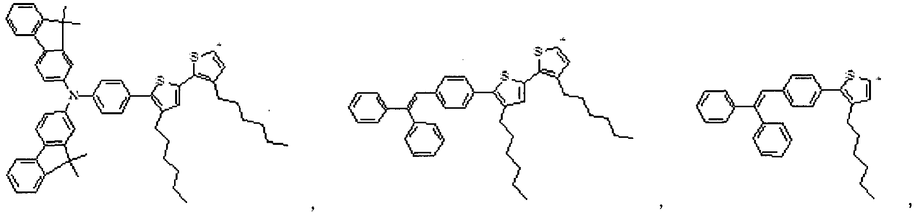 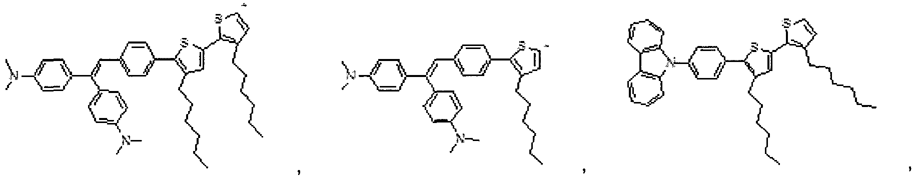 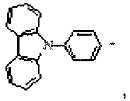 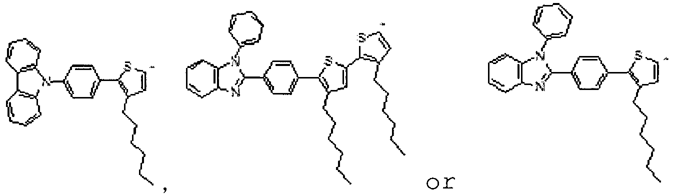 (here, R' is alkyl having 1 to 6 carbon atoms, n is an integer number from 0 to 5 and * is a coupling) independently, rings of aryl and heteroaryl groups have at least one substituent therein and the substituent may include an alkyl group, an alkoxy group, a halogen atom, an amide group, a cyano group, a hydroxyl group, a nitro group, an acyl group, an aryl group or heterogryl of C₁₋₃₀, independently, includes compounds according to the present invention.
[Item 3]
   The thienothiophene-based dye according to item 1, wherein the compound in the chemical formula 4 comprises one of compounds which are represented as compounds 142 to 157 below are embodiments of the present invention; 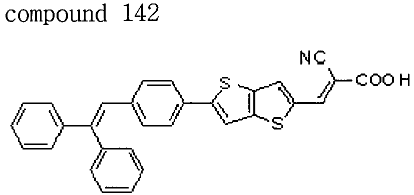 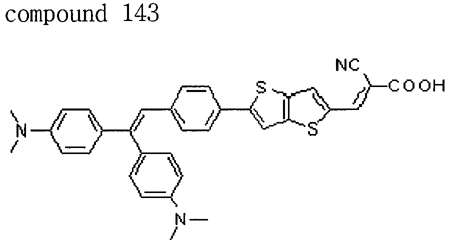 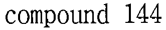 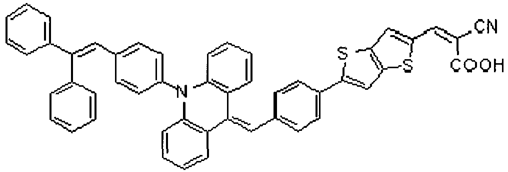 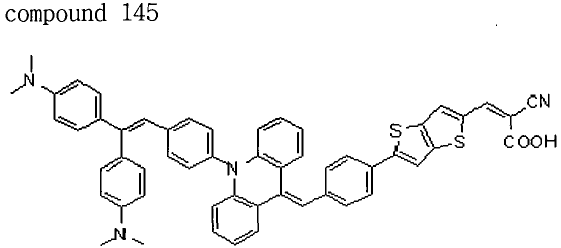 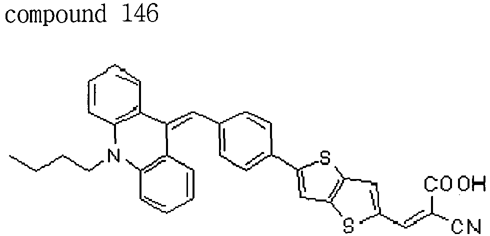 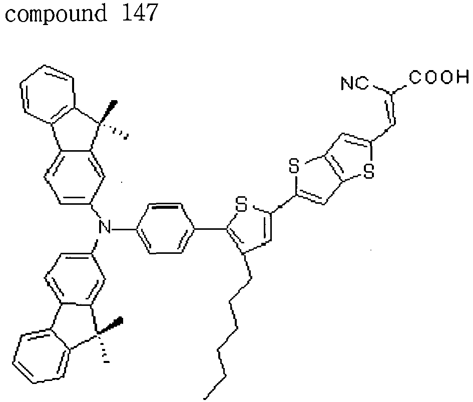 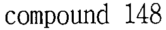 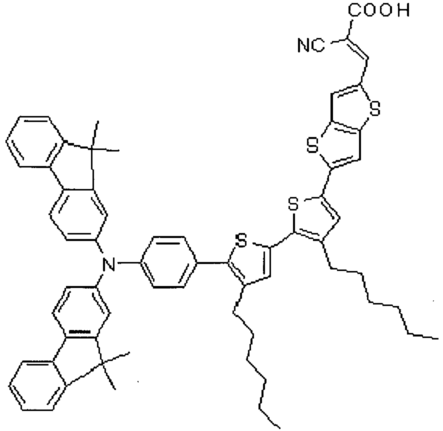 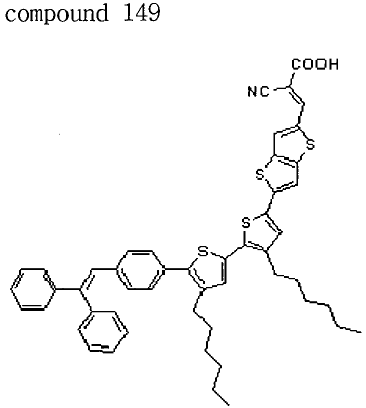 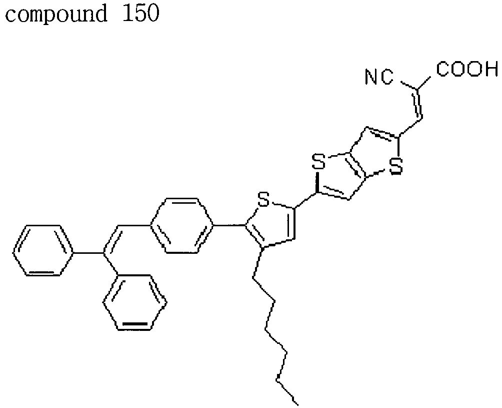 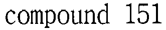 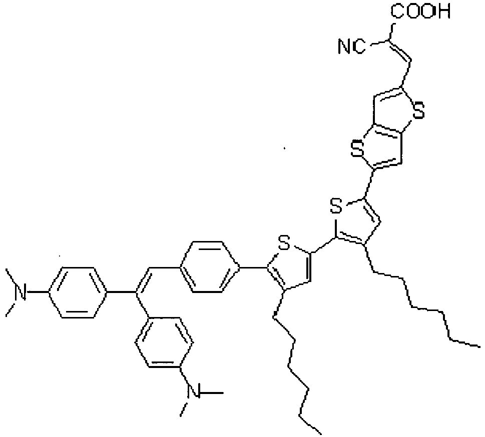 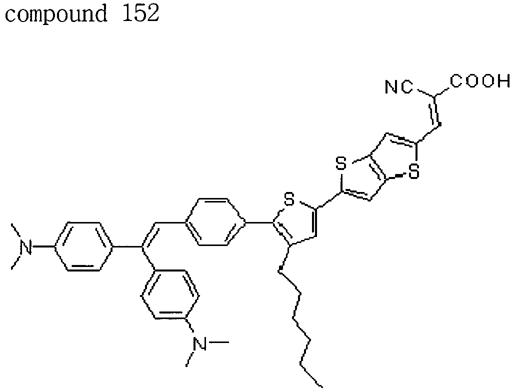 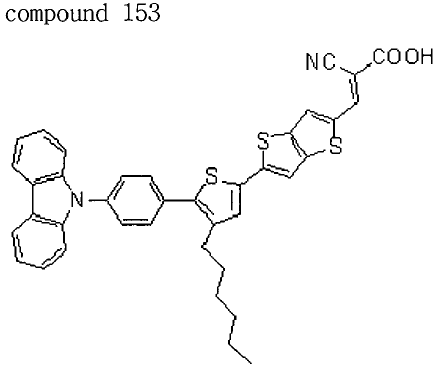 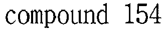 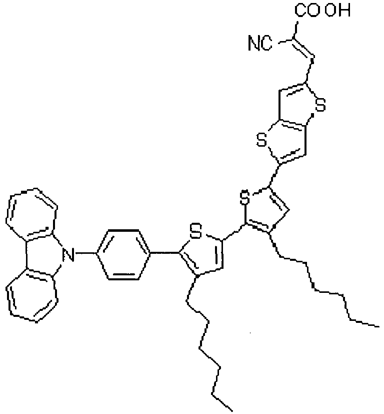 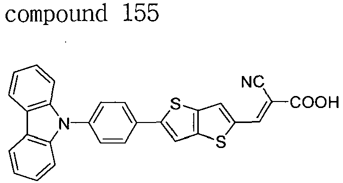 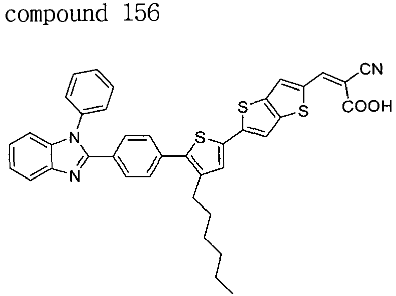 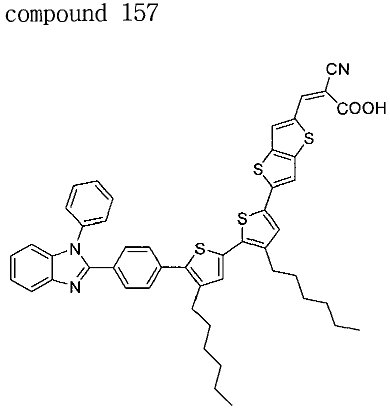
[Item 4]
   A preparation of a thienothiophene-based dye which is represented by a chemical formula 4 according to item 1, wherein,
   R₁ is 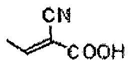 or 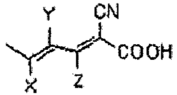 and X, Y and Z comprise hydrogen or alkyl, alkoxy or heteroalkyl of C₁₋₃₀;
   R₂ and R₃ comprise hydrogen, halogen, amide, cyano, hydroxyl, nitro, acyl, C₁₋₃₀ alkyl, C₁₋₃₀ alkoxy, C₁₋₃₀ alkylcarbonyl or C₆₋₂₀ aryl, independently, and these are coupled with each other to form an oxygen-containing hetero ring if they are C₁₋₃₀ alkoxy; and
   n is an integer number from 1 to 10; and
   Ar is 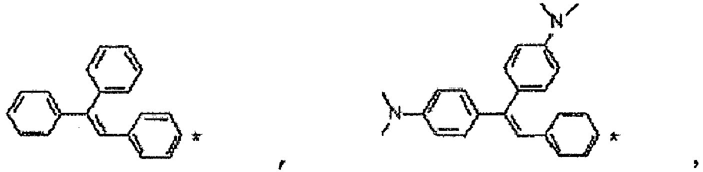 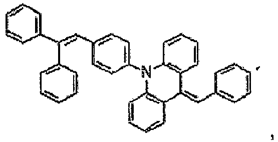 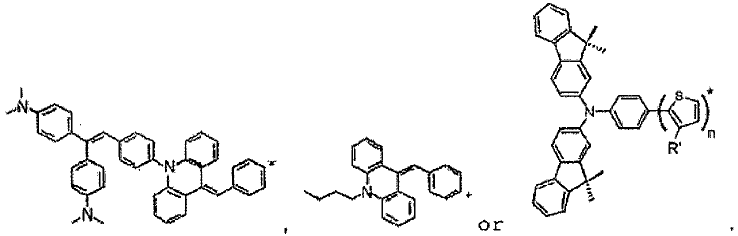 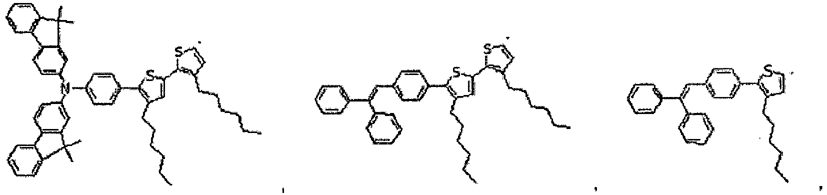 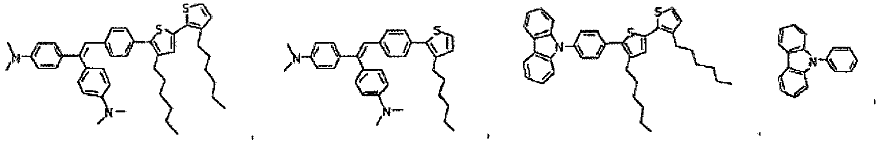 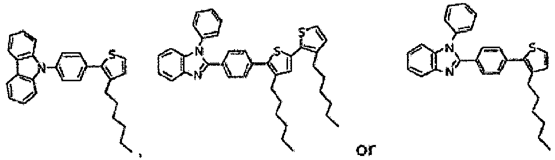 (here R' is alkyl having 1 to 6 carbon atoms, n is an integer number from 0 to 5 and * is a coupling)
   independently, rings of aryl and heteroaryl groups have at least one substituent therein and the substituent may include an alkyl group, an alkoxy group, a halogen atom, an amide group, a cyano group, a hydroxyl group, a nitro group, an acyl group, an aryl group or heteroaryl of C₁₋₃₀, independently,
   including a compound in a chemical formula 5 below reacting with n-butyllithium and then with a compound in a chemical formula 6 below:

   [Chemical formula 5] Ar-H₁

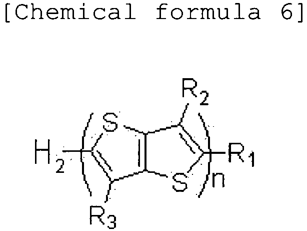

   wherein R₁, R₂, R₃, Ar and n in the chemical formulas 5 and 6 are as defined in the chemical formula 4 according to item 1, and H₁ and H₂ comprise halogen, independently.
[Item 5]
   A dye-sensitized photoelectric conversion element which comprises oxide semiconductor particles applied with the dye according to item 1.
[Item 6]
   The dye-sensitized photoelectric conversion element according to item 5, wherein the oxide semiconductor particles are applied with a thiophene-based dye under a presence of an inclusion compound.
[Item 7]
   The dye-sensitized photoelectric conversion element according to item 5, wherein the oxide semiconductor particles comprise titanium dioxide as a compulsory component.
[Item 8]
   The dye-sensitized photoelectric conversion element according to item 5, wherein the oxide semiconductor particles have an average diameter of 1 to 500nm.
[Item 9]
   A dye-sensitized solar cell which comprises the dye-sensitized photoelectric conversion element according to item 5 as an electrode.
[Item 10]
   The dye-sensitized solar cell according to item 9, which is prepared by an operation of coating TiO₂ paste on a transparent conductive substrate, an operation of firing the paste-coated substrate to form a TiO₂ layer, an operation of dipping the substrate having the TiO₂ layer into a solution including the dissolved dye represented by compounds 142 to 157 to form a TiO₂ film electrode having the dye, an operation of providing a second glass substrate including a counter electrode on the TiO₂ film electrode, an operation of forming a hole to pass through the second glass substrate and the counter electrode, an operation of coupling the counter electrode and the TiO₂ film electrode by heat and pressure, leaving a thermoplastic polymer film therebetween, an operation of injecting an electrolyte to the thermoplastic polymer film interposed between the counter electrode and the TiO₂ film electrode through the hole and an operation of sealing the thermoplastic polymer film.

## Claims

1. A thienothiophene-based dye comprising one of compounds which are represented as compounds 142 to 157 below;

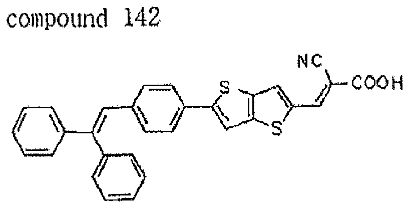

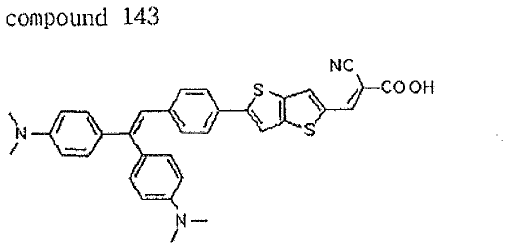

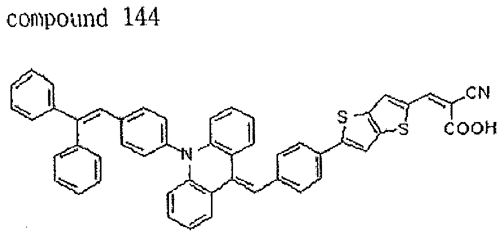

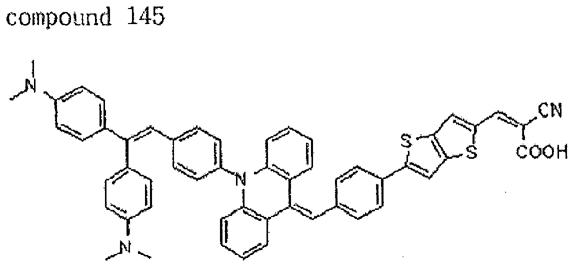

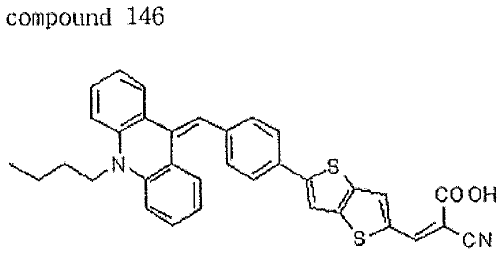

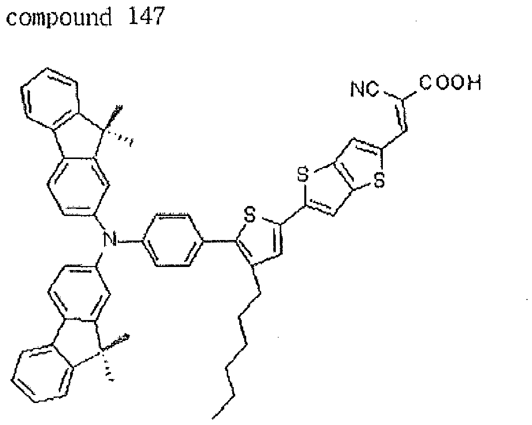

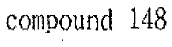

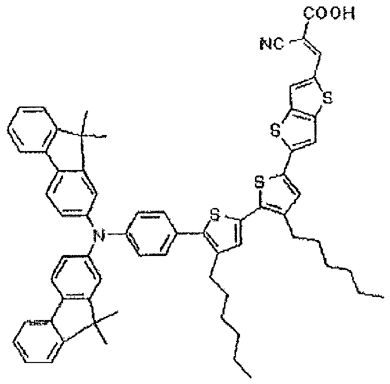

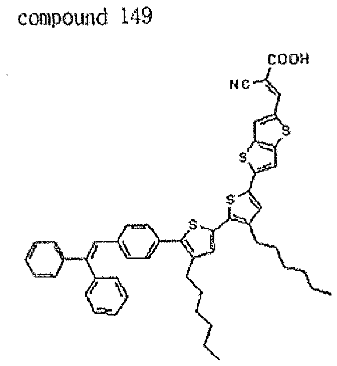

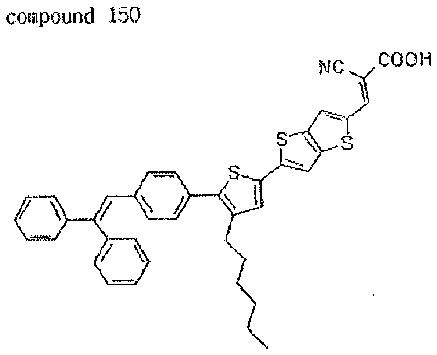

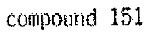

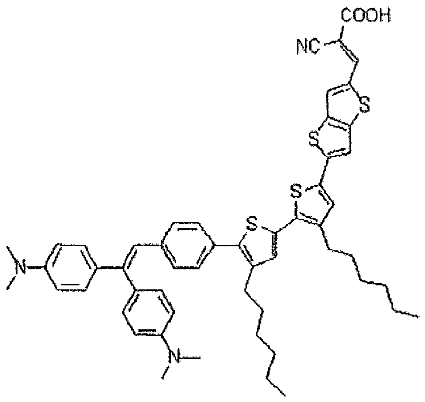

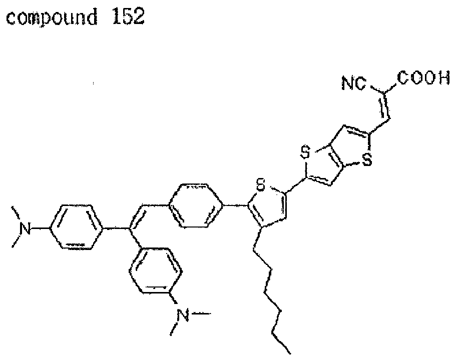

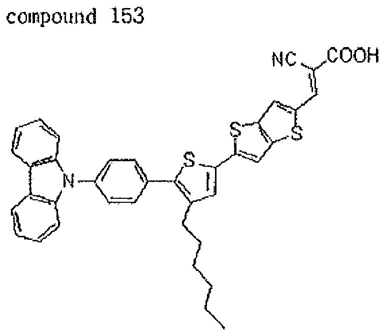

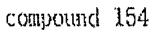

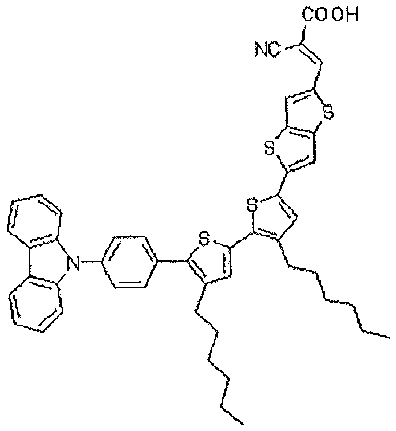

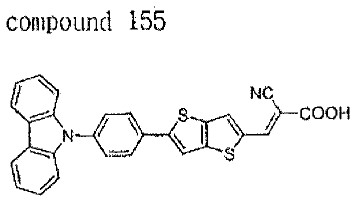

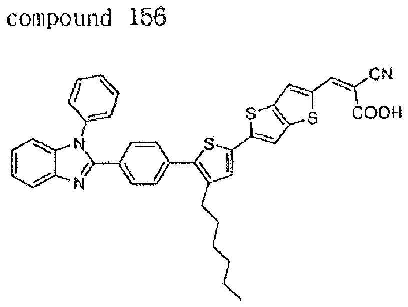

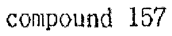

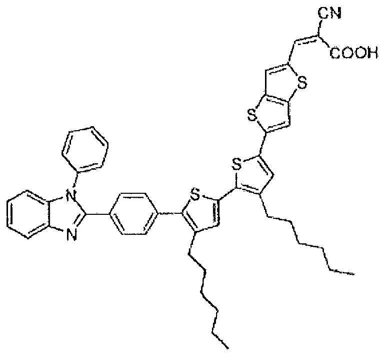

2. A preparation of a thienothiophene-based dye which is represented by a chemical formula 4 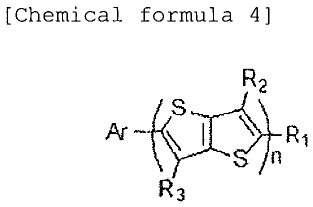 wherein,
R₁ is 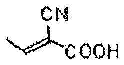 or 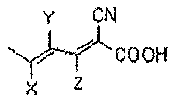 and X, Y and Z comprise hydrogen or alkyl, alkoxy or heteroalkyl of C₁₋₃₀;
R₂ and R₃ comprise hydrogen, halogen, amide, cyano, hydroxyl, nitro, acyl, C₁₋₃₀ alkyl, C₁₋₃₀ alkoxy, C₁₋₃₀ alkylcarbonyl or C₆₋₂₀ aryl, independently, and these are coupled with each other to form an oxygen-containing hetero ring if they are C₁₋₃₀ alkoxy; and
n is an integer number from 1 to 10; and
Ar is 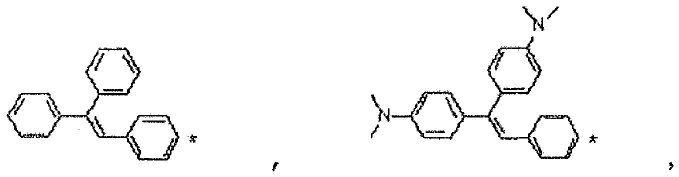 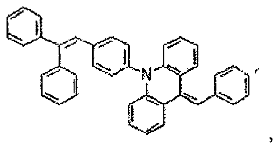 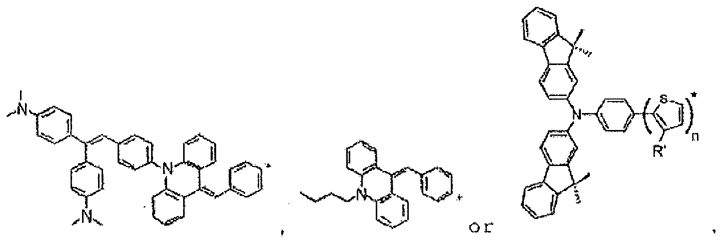 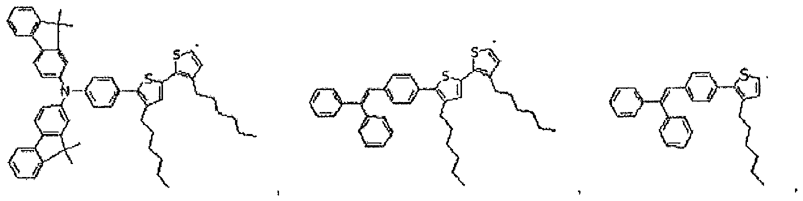 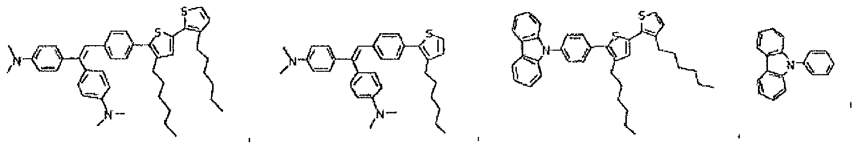 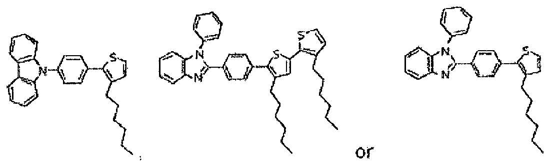 (here R' is alkyl having 1 to 6 carbon atoms, n is an integer number from 0 to 5 and * is a coupling) independently, rings of aryl and heteroaryl groups have at least one substituent therein and the substituent may include an alkyl group, an alkoxy group, a halogen atom, an amide group, a cyano group, a hydroxyl group, a nitro group, an acyl group, an aryl group or heteroaryl of C₁₋₃₀, independently,
including a compound in a chemical formula 5 below reacting with n-butyllithium and then with a compound in a chemical formula 6 below:
[Chemical formula 5] Ar-H₁ 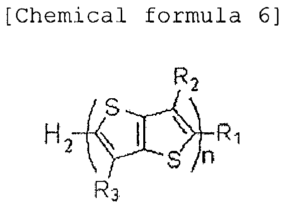
wherein R₁, R₂, R₃, Ar₁ and n in the chemical formulas 5 and 6 are as defined in the chemical formula 4, and H₁ and H₂ comprise halogen, independently.

3. A dye-sensitized photoelectric conversion element which comprises oxide semiconductor particles applied with the dye according to claim 1.

4. The dye-sensitized photoelectric conversion element according to claim 3, wherein the oxide semiconductor particles are applied with a thiophene-based dye under a presence of an inclusion compound.

5. The dye-sensitized photoelectric conversion element according to claim 3, wherein the oxide semiconductor particles comprise titanium dioxide as a compulsory component.

6. The dye-sensitized photoelectric conversion element according to claim 3, wherein the oxide semiconductor particles have an average diameter of 1 to 500nm.

7. A dye-sensitized solar cell which comprises the dye-sensitized photoelectric conversion element according to claim 3 as an electrode.

8. The dye-sensitized solar cell according to claim 7, which is prepared by an operation of coating TiO₂ paste on a transparent conductive substrate, an operation of firing the paste-coated substrate to form a TiO₂ layer, an operation of dipping the substrate having the TiO₂ layer into a solution including the dissolved dye to form a TiO₂ film electrode having the dye, an operation of providing a second glass substrate including a counter electrode on the TiO₂ film electrode, an operation of forming a hole to pass through the second glass substrate and the counter electrode, an operation of coupling the counter electrode and the TiO₂ film electrode by heat and pressure, leaving a thermoplastic polymer film therebetween, an operation of injecting an electrolyte to the thermoplastic polymer film interposed between the counter electrode and the TiO₂ film electrode through the hole and an operation of sealing the thermoplastic polymer film.

## Patentansprüche

1. Farbstoff auf Thienothiophenbasis, aufweisend eine der Verbindungen, die als nachstehende Verbindungen 142 bis 157 dargestellt sind:

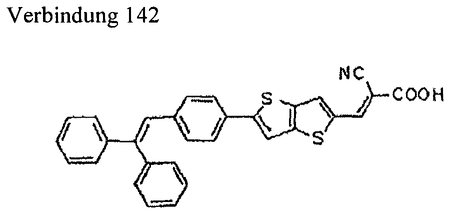

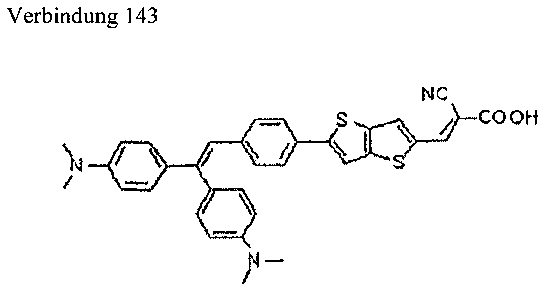

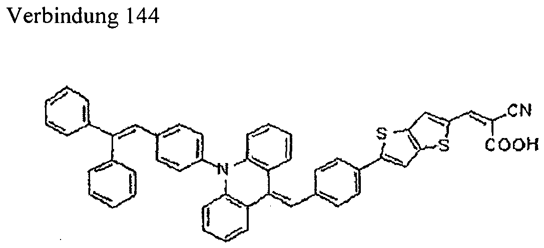

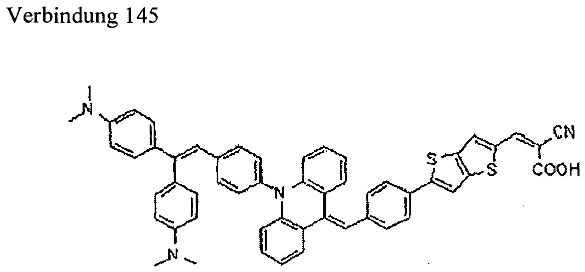

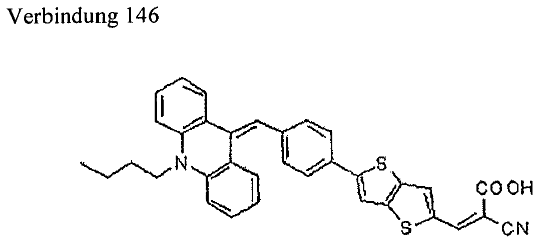

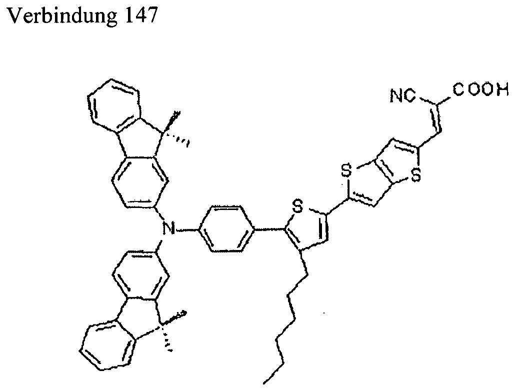

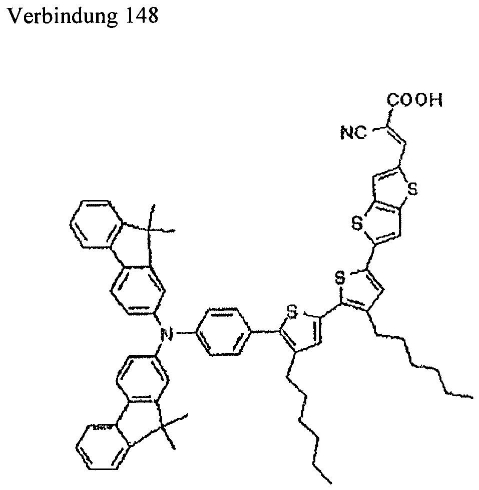

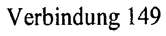

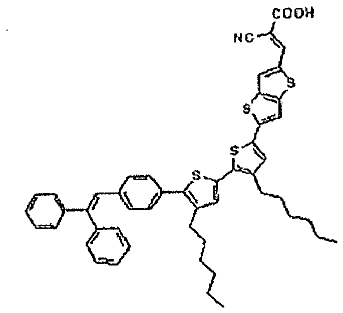

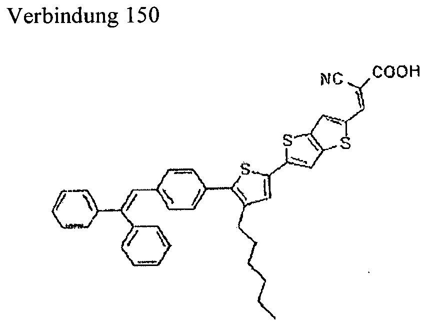

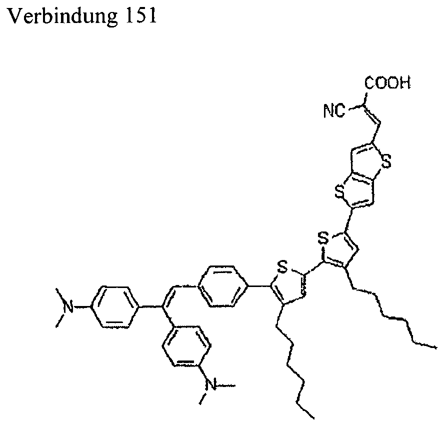

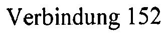

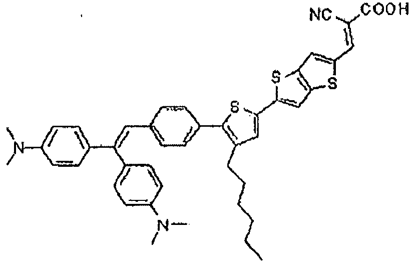

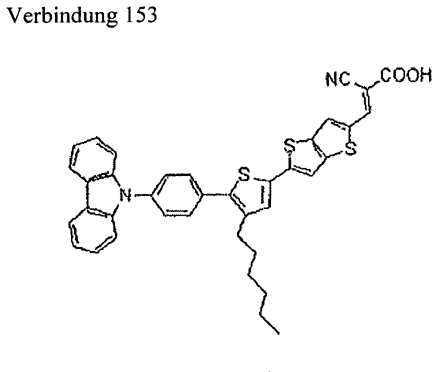

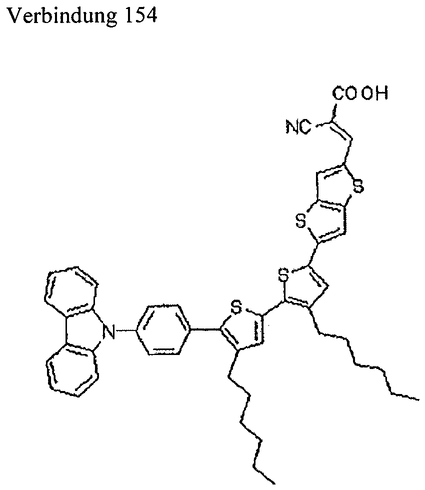

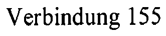

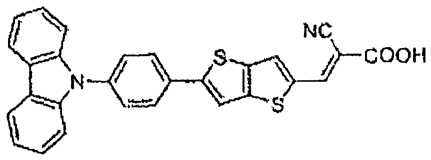

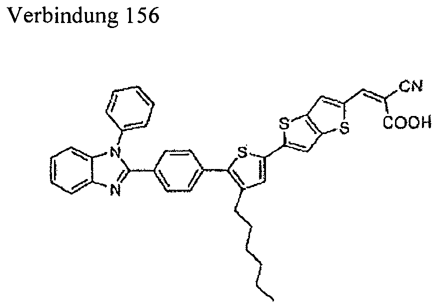

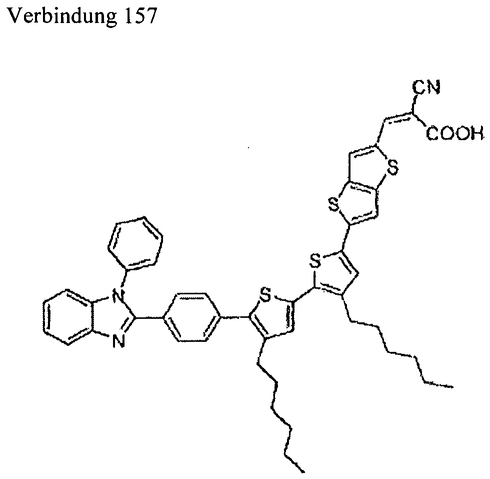

2. Herstellung eines Farbstoffs auf Thienothiophenbasis, der durch eine chemische Formel 4 dargestellt ist, 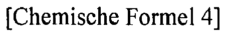 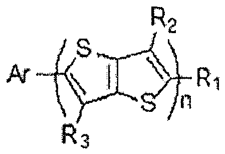 wobei
R₁ 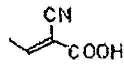 oder 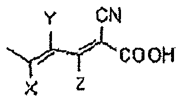 ist und X, Y und Z Wasserstoff oder Alkyl, Alkoxy oder Heteroalkyl von C₁₋₃₀ aufweisen;
R₂ and R₃ unabhängig Wasserstoff, Halogen, Amid, Cyano, Hydroxyl, Nitro, Acyl, C₁₋₃₀ Alkyl, C₁₋₃₀ Alkoxy, C₁₋₃₀ Alkylcarbonyl oder C₆₋₂₀ Aryl aufweisen, und diese miteinander verbunden sind, um einen Sauerstoff-haltigen Heteroring zu bilden, wenn sie C₁₋₃₀ Alkoxy sind; und
n eine ganze Zahl von 1 bis 10 ist; und
Ar 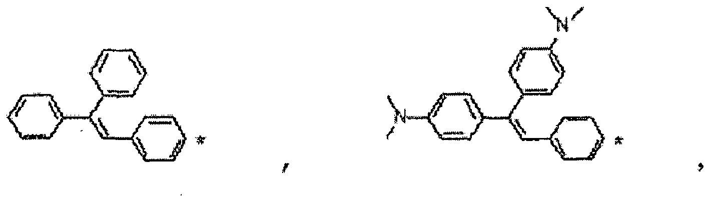 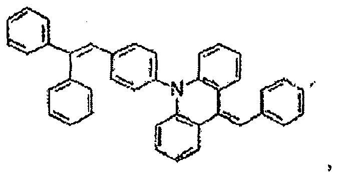 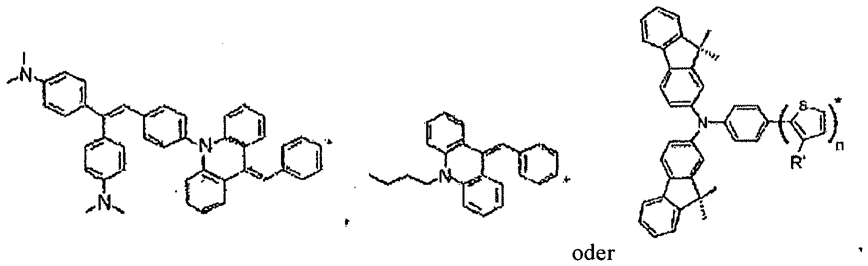 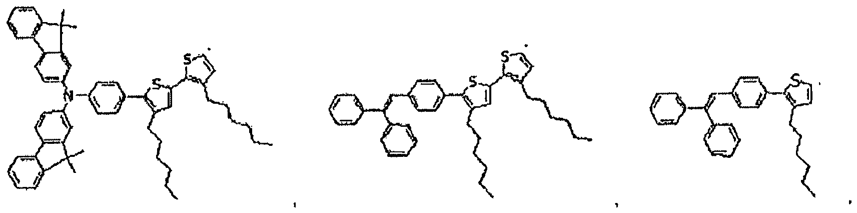 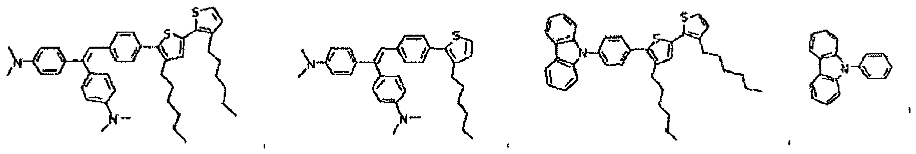 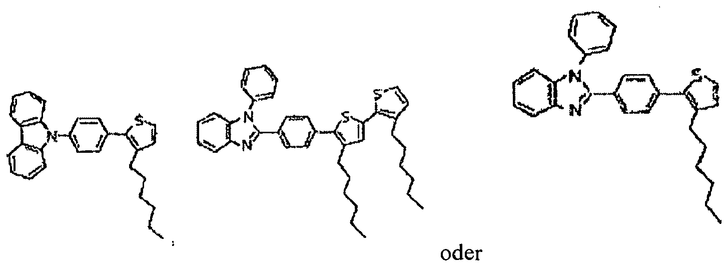 ist (hier ist R' Alkyl mit ,1 bis 6 Kohlenstoffatomen, n ist eine ganze Zahl von 0 bis 5 und * ist eine Bindung)
unabhängig Ringe von Aryl- und Heteroarylgruppen mindestens einen Substituenten darin aufweisen und der Substituent unabhängig eine Alkylgruppe, eine Alkoxygruppe, ein Halogenatom, eine Amidgruppe, eine Cyanogruppe, eine Hydroxylgruppe, eine Nitrogruppe, eine Acylgruppe, eine Arylgruppe oder Heteroaryl von C₁₋₃₀ einschließen kann,
einschließend Reagierenlassen einer Verbindung einer nachstehenden chemischen Formel 5 mit n-Butyllithium und dann mit einer Verbindung einer nachstehenden Formel 6:
[Chemische Formel 5] Ar-H₁ 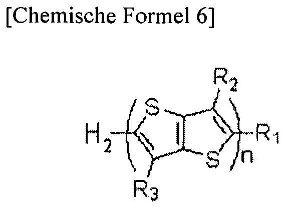
wobei R₁, R₂, R₃, Ar₁ und n in den chemischen Formeln 5 und 6 wie in der chemischen Formel 4 definiert sind und H₁ und H₂ unabhängig Halogen aufweisen.

3. Farbstoffsensibilisiertes photovoltaisches Umwandlungselement, das mit dem Farbstoff gemäß Anspruch 1 aufgetragene Oxid-Halbleiter-Partikel aufweist.

4. Farbstoffsensibilisiertes photovoltaisches Umwandlungselement gemäß Anspruch 3,
wobei die Oxid-Halbleiter-Partikel mit einem Farbstoff auf Thiophenbasis in Gegenwart einer Einschlussverbindung aufgetragen sind.

5. Farbstoffsensibilisiertes photovoltaisches Umwandlungselement gemäß Anspruch 3,
wobei die Oxid-Halbleiter-Partikel Titandioxid als eine wesentliche Komponente aufweisen.

6. Farbstoffsensibilisiertes photovoltaisches Umwandlungselement gemäß Anspruch 3,
wobei die Oxid-Halbleiter-Partikel einen durchschnittlicher Durchmesser von 1 bis 500 nm aufweisen.

7. Farbstoffsensibilisierte Solarzelle, die das farbstoffsensibilisierte photovoltaische Umwandlungs-element gemäß Anspruch 3 als eine Elektrode aufweist.

8. Farbstoffsensibilisierte Solarzelle gemäß Anspruch 7, die hergestellt ist durch einen Vorgang des Auftragens einer TiO₂-Paste auf ein transparentes leitfähiges Substrat, einen Vorgang des Feuerns des Pasten-beschichteten Substrats zur Bildung einer TiO₂-Schicht, einen Vorgang des Tauchens des Substrats mit der TiO₂-Schicht in eine Lösung, die den gelösten Farbstoff enthält, um eine TiO₂-Filmelektrode mit dem Farbstoff zu bilden, einen Vorgang des Bereitstellens eines zweiten Glassubstrats mit einer Gegenelektrode auf der TiO₂-Filmelektrode, einen Vorgang des Bildens eines Lochs zum Verlaufen durch das zweite Glassubstrat und die Gegenelektrode, einen Vorgang des Verbindens der Gegenelektrode und der TiO₂-Filmelektrode durch Wärme und Druck, wobei ein thermoplastischer Polymerfilm dazwischen hinterlassen wird, einen Vorgang des Spritzens eines Elektrolyts zu dem thermoplastischen Polymerfilm, der zwischen der Gegenelektrode und der TiO₂-Filmelektrode liegt, durch das Loch und einen Vorgang des Abdichtens des thermoplastischen Polymerfilms.

## Revendications

1. Colorant à base de thiénothiophène comprenant l'un des composés qui sont représentés comme les composés 142 à 157 ci-dessous :

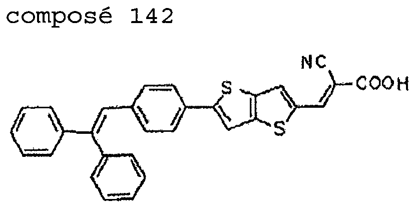

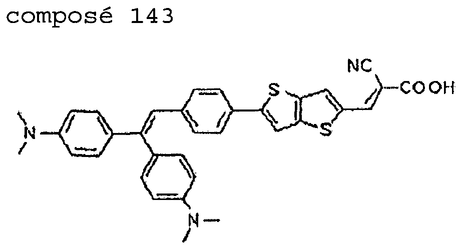

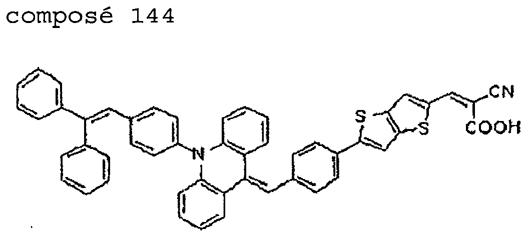

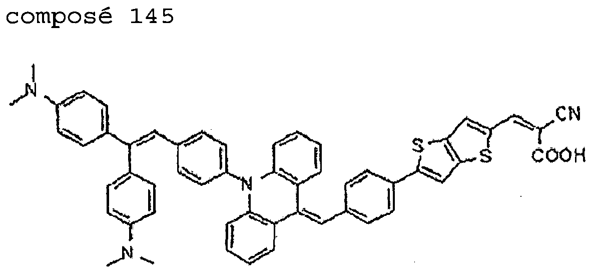

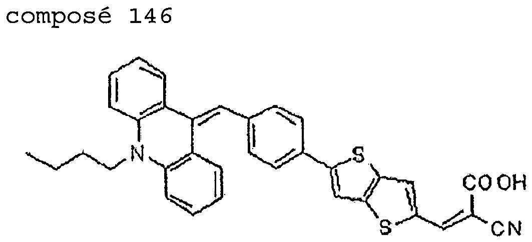

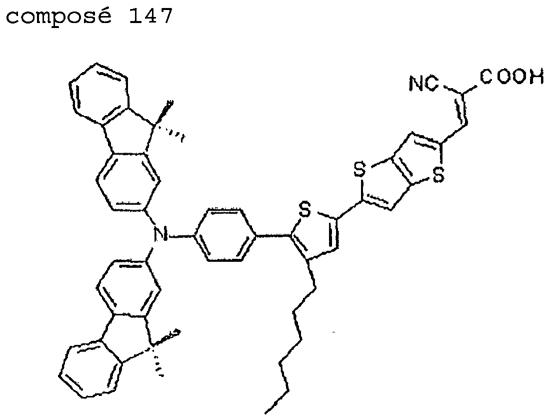

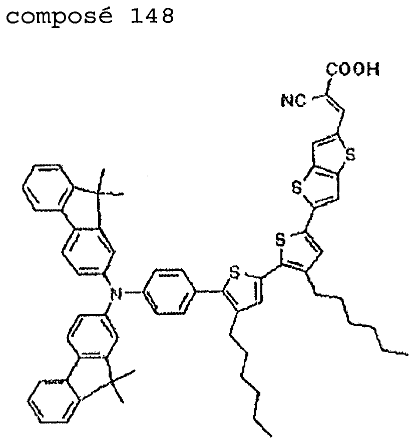

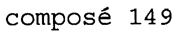

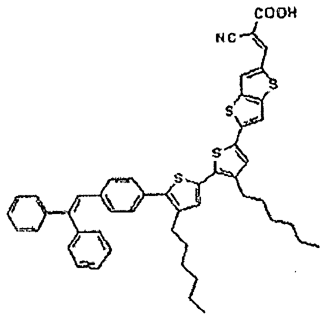

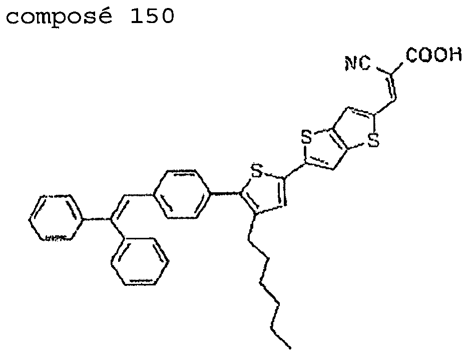

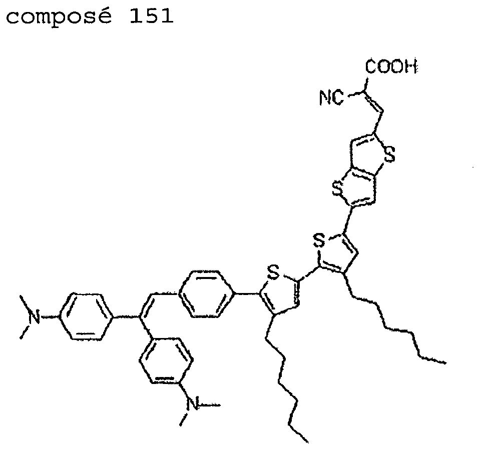

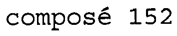

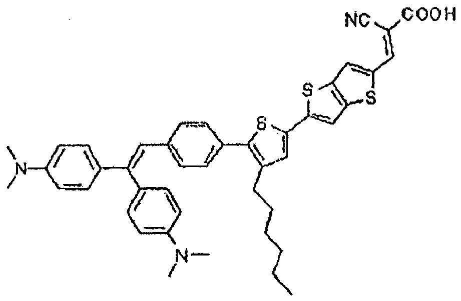

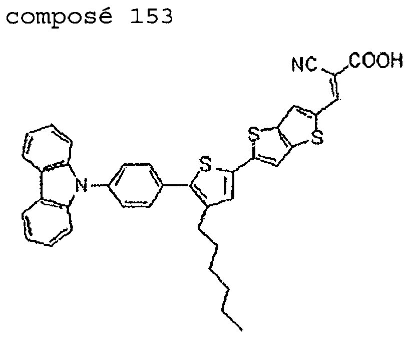

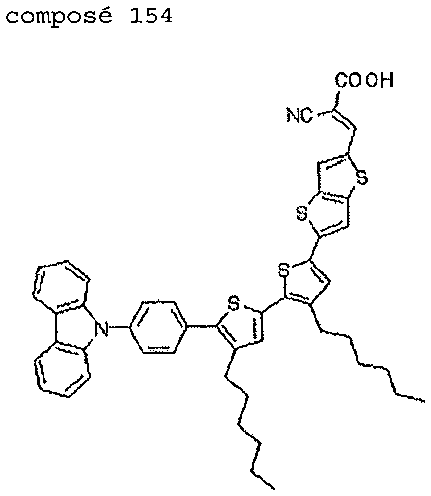

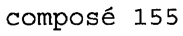

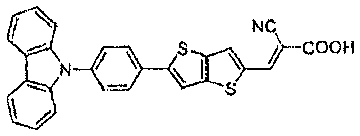

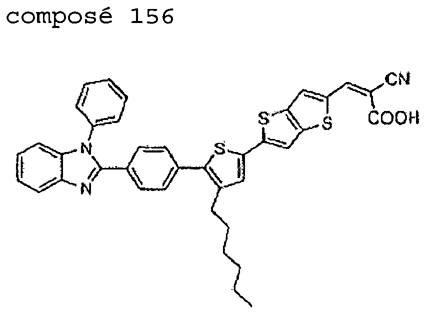

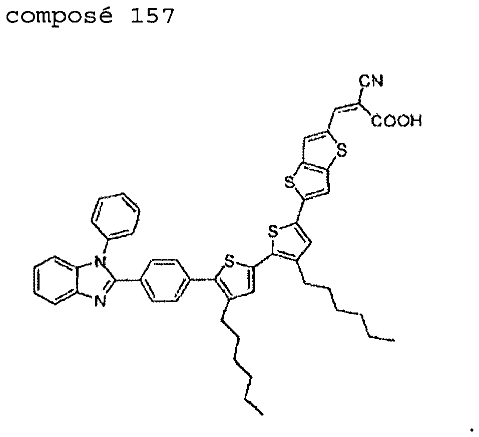

2. Préparation d'un colorant à base de thiénothiophène qui est représenté par une formule chimique 4 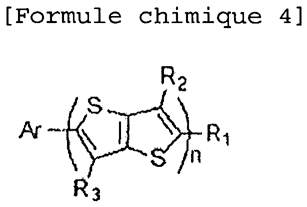 dans laquelle,
R₁ est 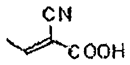 ou 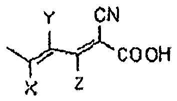 et X, Y et Z comprennent hydrogène ou alkyle, alcoxy ou hétéroaryle en C₁₋₃₀ ;
R₂ et R₃ comprennent indépendamment hydrogène, halogène, amide, cyano, hydroxyle, nitro, acyle, alkyle en C₁₋₃₀, alcoxy en C₁₋₃₀, alkylcarbonyle en C₁₋₃₀ ou aryle en C₁₋₃₀, et ils sont couplés les uns aux autres pour former un hétérocycle contenant de l'oxygène s'il sont un alcoxy en C₁₋₃₀ ; et
n est un nombre entier de 1 à 10 ; et
Ar est indépendamment l'un de l'autre 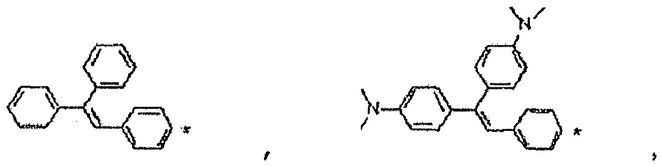 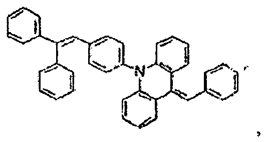 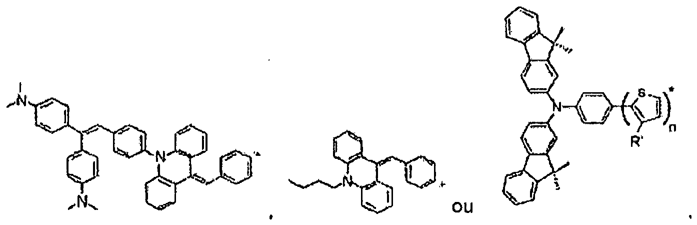 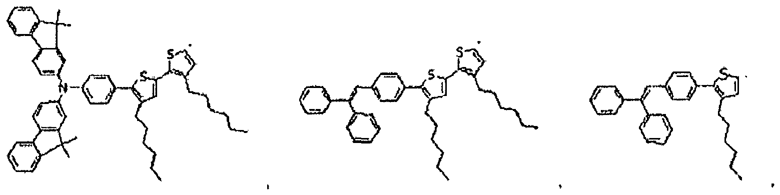 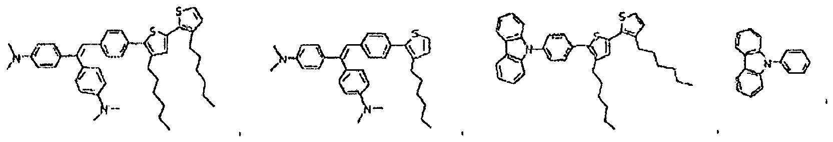 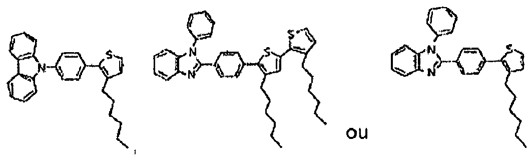 (ici R' est alkyle ayant 1 à 6 atomes de carbone, n est un nombre entier de 0 à 5 et * est un couplage),
des cycles de groupes aryle et hétéroaryle ont au moins un substituant et le substituant peut inclure indépendamment l'un de l'autre un groupe alkyle, un groupe alcoxy, un atome d'halogène, un groupe amide, un groupe cyano, un groupe hydroxyle, un groupe nitro, un groupe acyle, un groupe aryle ou hétéroaryle en C₁₋₃₀,
incluant un composé dans une formule chimique 5 ci-dessous réagissant avec du n-butyllithium puis avec un composé dans une formule chimique 6 ci-dessous :
[Formule chimique 5] Ar-H₁ 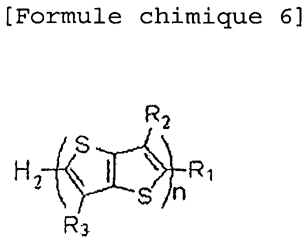
dans laquelle R₁, R₂, R₃, Ar₁ et n dans les formules chimiques 5 et 6 sont tels que définis dans la formule chimique 4, et H₁ et H₂ comprennent indépendamment l'un de l'autre un halogène.

3. Elément de conversion photoélectrique sensibilisé par colorant qui comprend des particules de semi-conducteur à oxyde auxquelles est appliqué le colorant selon la revendication 1.

4. Elément de conversion photoélectrique sensibilisé par colorant selon la revendication 3, dans lequel les particules de semi-conducteur à oxyde auxquelles est appliqué un colorant à base de thiophène en présence d'un composé d'inclusion.

5. Elément de conversion photoélectrique sensibilisé par colorant selon la revendication 3, dans lequel les particules de semi-conducteur à oxyde comprennent du dioxyde de titane en tant que composant obligatoire.

6. Elément de conversion photoélectrique sensibilisé par colorant selon la revendication 3, dans lequel les particules de semi-conducteur à oxyde ont un diamètre moyen de 1 à 500 nm.

7. Cellule solaire sensibilisée par colorant comprenant l'élément de conversion photoélectrique sensibilisé par colorant selon la revendication 3 en tant qu'électrode.

8. Cellule solaire sensibilisée par colorant selon la revendication 7, qui est préparée par une opération de revêtement de pâte de TiO₂ sur un substrat conducteur transparent, une opération de mise à feu du substrat revêtu de pâte pour former une couche de TiO₂, une opération de plongement du substrat comportant la couche de TiO₂ dans une solution incluant le colorant dissous pour former une électrode à film de TiO₂ comportant le colorant, une opération de fourniture d'un second substrat de verre incluant une contre-électrode sur l'électrode à film de TiO₂, une opération de formation d'un trou pour traverser le second substrat de verre et la contre-électrode, une opération de couplage de la contre-électrode et de l'électrode à film de TiO₂ par chaleur et pression, laissant un film polymère thermoplastique entre elles, une opération d'injection d'un électrolyte dans le film polymère thermoplastique interposé entre la contre-électrode et l'électrode à film de TiO₂ à travers le trou et une opération de scellage du film polymère thermoplastique.
